# EUROPÄISCHE PATENTSCHRIFT

(11) **EP 1 059 931 B1**
(45) Veröffentlichungstag und Bekanntmachung des Hinweises auf die Patenterteilung: **27.11.2002**
(21) Anmeldenummer: 99910331.0
(22) Anmeldetag: 05.03.1999
(51) Int. Cl.: A61K 38/17, C12N 5/00, A61K 48/00, A61P 43/00

(54) **VERWENDUNG VON CD137 ZUR FÖRDERUNG DER PROLIFERATION PERIPHERER MONOCYTEN**
UTILIZATION OF CD137 IN ORDER TO PROMOTE THE PROLIFERATION OF PERIPHERAL MONOCYTES
UTILISATION DU FACTEUR CD137 POUR LA STIMULATION DE LA PROLIFERATION DE MONOCYTES PERIPHERIQUES

(30) Priorität: 15.07.1998 EP 98103859
(43) Veröffentlichungstag der Anmeldung: 20.12.2000
(73) Patentinhaber: MERCKLE GMBH, D-89079 Ulm (DE)
(72) Erfinder: SCHWARZ, Herbert, D-92245 Theuern (DE); LANGSTEIN, Joachim, D-93053 Regensburg (DE)
(74) Vertreter: Kinzebach, Werner, Dr.
(86) Internationale Anmeldenummer: EP9901440
(87) Internationale Veröffentlichungsnummer: WO99044629

(56) Entgegenhaltungen:
- EP-A- 0 426 458
- WO-A-94/26290
- CA-A- 2 108 401
- LANGSTEIN, JOACHIM (1) ET AL: "CD137, a member of the TNF receptor family regulates monocyte activation and survival via reverse signaling." CANCER GENE THERAPY, (NOV.-DEC., 1997) VOL. 4, NO. 6 CONF. SUPPL., PP. S58. MEETING INFO.: SIXTH INTERNATIONAL CONFERENCE ON GENE THERAPY OF CANCER SAN DIEGO, CALIFORNIA, USA NOVEMBER 20-22, 1997 , XP002122039
- SHUFORD W W ET AL: "4 - 1BB costimulatory signals preferentially induce CD8+ T cell proliferation and lead to the amplification in vivo of cytotoxic T cell responses." JOURNAL OF EXPERIMENTAL MEDICINE, (1997 JUL 7) 186 (1) 47-55. , XP002122040
- XING Z ET AL: "Human upper airway structural cell-derived cytokines support human peripheral blood monocyte survival: a potential mechanism for monocyte/macrophage accumulation in the tissue." AMERICAN JOURNAL OF RESPIRATORY CELL AND MOLECULAR BIOLOGY, (1992 FEB) 6 (2) 212-8. , XP002122041 in der Anmeldung erwähnt
- SCHWARZ H ET AL: "A receptor induced by lymphocyte activation ( ILA ): a new member of the human nerve-growth-factor/tumor-necrosis-factor receptor family." GENE, (1993 DEC 8) 134 (2) 295-8. , XP002122042 in der Anmeldung erwähnt
- LANGSTEIN J ET AL: "CD137 ( ILA /4-1BB), a member of the TNF receptor family, induces monocyte activation via bidirectional signaling." JOURNAL OF IMMUNOLOGY, (1998 MAR 1) 160 (5) 2488-94. , XP002122043 in der Anmeldung erwähnt
- SCHWARZ H ET AL: "ILA, a member of the human nerve growth factor/tumor necrosis factor receptor family, regulates T-lymphocyte proliferation and survival." BLOOD, (1996 APR 1) 87 (7) 2839-45. , XP002122044 in der Anmeldung erwähnt

## Beschreibung

Die vorliegende Erfindung betrifft die Verwendung des Monozytenwachstumsfaktor CD137 zur Förderung der Proliferation peripherer Monozyten und insbesondere die Anwendung von CD137 zur Behandlung verschiedener Krankheitszustände, welche durch die proliferationsfördernde Wirkung von CD137 in neuartiger Weise therapierbar sind.

Periphere Monozyten des Blutes sowie die aus ihnen hervorgehenden Makrophagen aus Körperhöhlen und Geweben sind Bestandteil des mononukleären Phagozytensystems des Körpers. Insbesondere stellen Monozyten und Makrophagen Effektorzellen des unspezifischen Immunabwehrsystems des Körpers dar. Monozyten entwickeln sich über mehrere Zwischenstufen aus den hämatopoetischen Stammzellen im Knochenmark. Sie zirkulieren über einen Zeitraum von etwa 20 bis 30 Stunden im Blut. Von dort aus wandern sie in die verschiedenen Organe und Gewebesysteme aus und entwickeln sich dort zu ortsspezifischen Makrophagen. Dabei werden sie durch das umliegende Gewebe geprägt und entwickeln zusätzliche Funktionen. Man unterscheidet deshalb je nach Gewebetyp beispielsweise Makrophagen der Lunge (Alveolarmakrophagen), der Bauchhöhle (Peritonealmakrophagen), der Milz (Milzmakrophagen), der Leber (Kupffer-Zellen), der Gelenke, des Knochens (Osteoklasten), des Bindegewebes, des Gehirns und der Niere.

Monozyten und Makrophagen besitzen eine zentrale Stellung im Rahmen der im Körper ablaufenden Entzündungsreaktionen. In nicht-entzündetem Gewebe besteht die Aufgabe von Makrophagen in der Beseitigung gealteter Zellen. Außerdem produzieren sie eine große Anzahl löslicher Faktoren, die für die Kommunikation innerhalb des Immunsystems von Bedeutung sind. Sind die Zellen jedoch an einem Entzündungsprozeß beteiligt, so befinden sie sich in einem aktivierten Zustand mit stark veränderten phänotypischen und funktionellen Eigenschaften. Dabei üben sie wichtige, den Krankheitsablauf beeinflussende Effektorfunktionen aus. Dazu gehören die Phagozytose und intrazelluläre Abtötung von Mikroorganismen, Immunkomplexen und geschädigten Zellen, aber auch die antikörperabhängigen und - unabhängigen Zytotoxizitätsreaktionen gegen Tumorzellen, Parasiten und Virus-infizierten Zellen. Außerdem sind Monozyten und Makrophagen von zentraler Bedeutung bei der Induktion und Regulation der Immunantwort. Es handelt sich nämlich außerdem um sekretorisch hochaktive Zellen, die durch die vermehrte Freisetzung von Zytokinen die Immunantwort beeinflussen.

CD137 ist ein Mitglied der Tumornekrosefaktor-Rezeptorfamilien und ist außerdem unter den Bezeichnungen ILA oder 4-1BB (Homologes aus der Maus) bekannt (Kwon, B.S., et al, Proc Natl Acad Sci USA 86:1963, 1989; Schwarz H. et al, Gene 134:295,1993; Alderson, M.R., et al, Eur J Immunol 24:2219,1994). CD137 wird von aktivierten Lymphozyten und Monozyten exprimiert, wobei die Expression durch Primärzellen von einer Aktivierung abhängig ist (Schwarz H. et al, Blood 85:1043, 1995). Die CD137 Expression ist beispielsweise durch Aktivierung von T-Lymphozyten mit Phytohämagglutinin (PHA) oder Phorbol-12-myristat-13-acetat (PMA) rasch induzierbar. In Monocyten ist CD137 durch Aktivierung mit Lipopolysaccharid (LPS), IL-1β und PMA induzierbar. In B-Lymphozyten wird CD137-Expression durch Antikörper gegen Zelloberflächeimmuoglobulin oder TMA sowie durch Transformation mit EBV (Epstein Barr-Virus) induziert. In nicht-lymphoiden Zellen (wie insbesondere Chondrozyten) ist CD137 durch das proinflammatorische Cytokin IL-1β stark induzierbar. Lösliche Formen von CD137 werden durch differentielles Splicing erzeugt und können in erhöhten Konzentrationen in Seren von Patienten mit rheumatoider Arthritis nachgewiesen werden (Michel, J., et al, Eur J Immunol 28:290, 1998). Das Gen für menschliches CD137 sitzt auf Chromosom 1p36 in einem Cluster verwandter Gene (Schwarz, H., et al, Biochem Biophys Res Com 235:699, 1997).

Die WO-A-94/26290 beschreibt den Zelloberflächenrezeptor 4-1BB und den korrespondierenden Liganden davon.

Die EP-A-0 426 458 beschreibt einen Wachstumsfaktor, der aus der humanen Lebertumor Zelllinie T3M-30Lu gewonnen wird und die Proliferation peripherer Monozyten stimuliert, sowie dessen Verwendung zur Förderung der Immunabwehr.

Von rekombinantem CD137 Protein ist außerdem bekannt, daß es in immobilisierter Form eine Aktivierung von Monozyten bewirkt. Folge der Aktivierung ist eine verstärkte Expression pro- inflammatorischer Zytokine, eine Inhibition des anti-inflammatorischen Zytokins IL-10 sowie die Induktion von Aktivierungsmarken, wie ICAM (Langstein J. et al, J Immunol 160-2488, 1998). Eine lebensverlängernde oder proliferationsfördernde Wirkung auf Monozyten wird darin nicht beschrieben.

Wie bereits oben erwähnt, besitzen Monozyten eine Schlüsselfunktion im Rahmen der Immunantwort, und sind von essentieller Bedeutung für die Erzeugung gutartiger Immunreaktionen gegen Tumoren und Pathogene. Angezogen von Signalen, wie z.B. von Zytokinen, wandern sie aus dem Blutkreislauf an den Ort der Entzündung. Eine Akkumulation von Monozyten und Makrophagen stellt ein charakteristisches Merkmal chronischer Entzündungen dar. Diese Akkumulation wird durch Zytokine weiter gefördert, welche am Entzündungsort freigesetzt werden, wie z.B. Makrophagen-Kolonie-stimulierender Faktor (M-CSF), Granulozyten-Makrophagen-Kolonie-stimulierender Faktor (GM-CSF) und Interleukin 3 (IL-3), welche sich günstig auf das Überleben von Monozyten und Makrophagen auswirken (Young, D.A., J. Immunol 145:607, 1990; Xing, Z., et al, Am J Respir Cell Mol Biol 6:212, 1992; Bratton, D.L., et al, J Clin Invest 95:211, 1995).

Von peripheren Monozyten und Makrophagen wurde bisher angenommen, daß sie zur Proliferation, d.h. zur Vermehrung, nicht befähigt sind (vgl. Xing, Z. et al, Supra; van Furth, R., et al, Blood 54-485, 1979).

Aus obigen Ausführungen wird ersichtlich, daß die Behandlung von zahlreichen Erkrankungen, wie z.B. Tumoren, Infektionen durch Bakterien, Pilze oder Viren, deutlich verbessert werden könnte, wenn durch Vermehrung der Monozyten/Makrophagen eine verstärkte Phagozytone und intrazelluläre Abtötung von Mikroorganismen, Immunkomplexen und geschädigten Zellen sowie auch eine verbesserte Antikörper-abhängige oder -unabhängige Zytotoxizitätsreaktion gegenüber Tumorzellen, Mikroorganismen und davon infizierten Zellen herbeigeführt werden könnte.

Außerdem ist bekannt, daß verschiedene therapeutische Behandlungsformen, wie z.B. die Chemo- oder Strahlentherapie von Krebspatienten, sowie die Gabe von Immunsuppressiva. die Zahl von Monozyten und Makrophagen drastisch verringert. Nach Beendigung derartiger Therapieverfahren ist es in der Regel wünschenswert, die Zahl der Monozyten/Makrophagen wieder auf Werte im Normalbereich zu erhöhen und folglich das Immunsystem des Patienten wieder zu stabilisieren.

Es ist deshalb Aufgabe der vorliegenden Erfindung, einen Weg bereitzustellen, der es ermöglicht, die Zahl peripherer Monozyten und folglich die Zahl von Makrophagen gezielt zu erhöhen, um eine verbesserte Therapierung von Krankheitszuständen zu ermöglichen, welche mit einer nicht-ausreichenden Zahl aktiver Monozyten/Makrophagen assoziiert sind.

Diese Aufgabe konnte überraschenderweise ausgehend von der Feststellung gelöst werden, daß das an sich bekannte Zelloberflächenprotein CD137 und funktionale Analoga davon, entgegen bisheriger Annahmen, die Proliferation von peripheren Monozyten unabhängig von den hämatopoetischen Stammzellen induziert. Dies eröffnet überraschenderweise eine Vielzahl neuer therapeutischer Anwendungsmöglichkeiten für CD137.

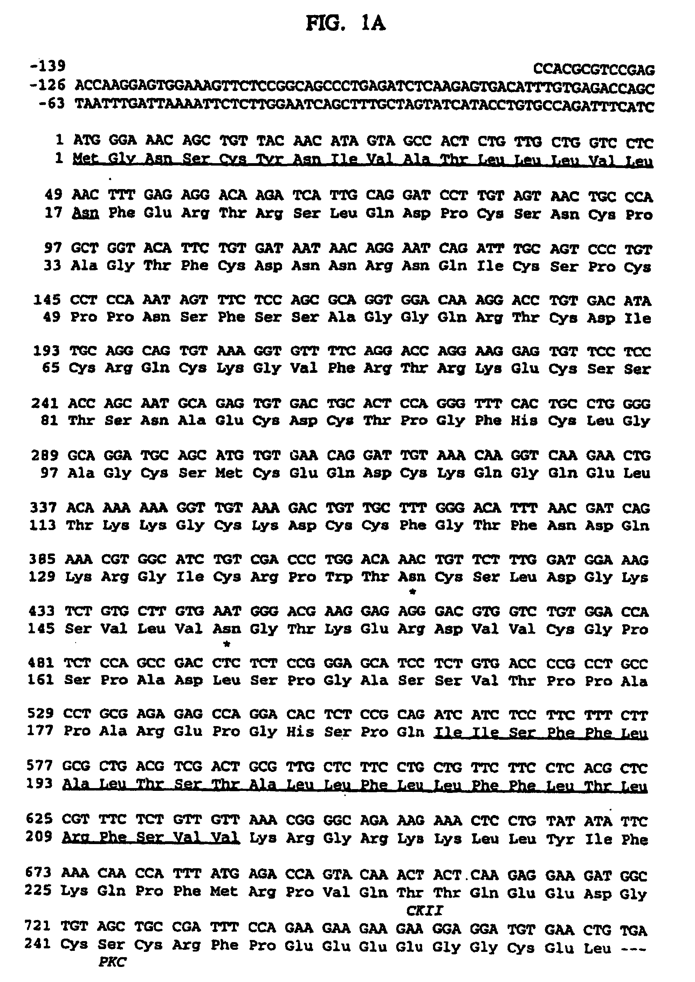

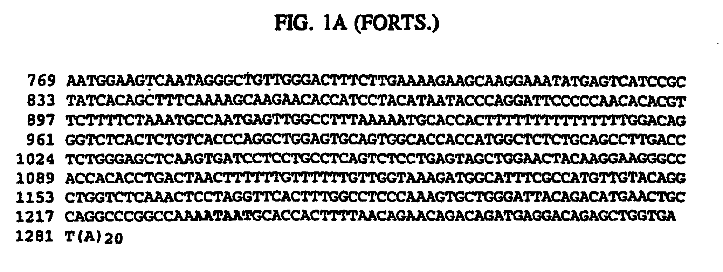

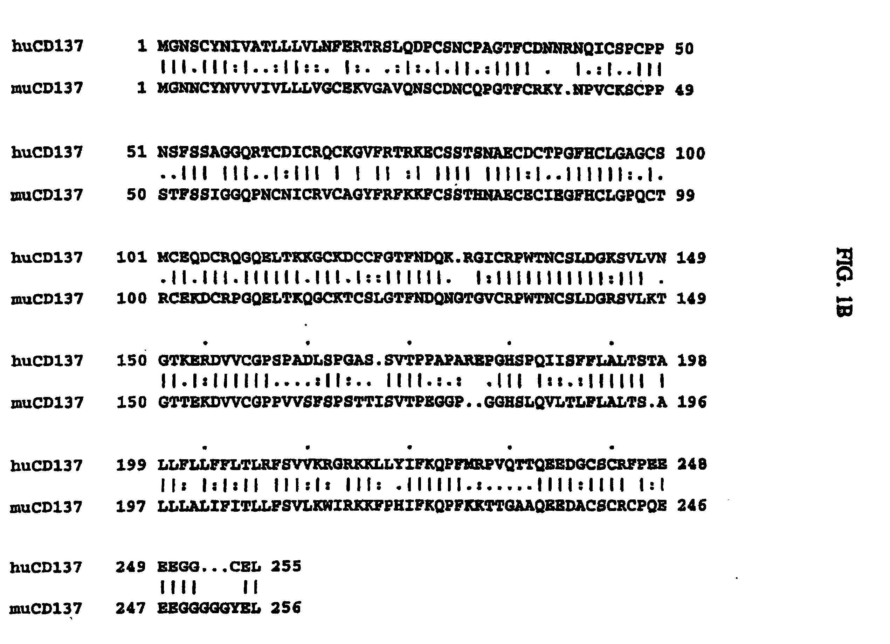

Spezielle Ausführungsformen der vorliegenden Erfindung werden im folgenden unter Bezugnahme auf die beiliegenden Figuren näher erläutert. Dabei zeigt
- Fig. 1: (A) die cDNA-Sequenz und die davon abgeleitete Aminosäuresequenz von humanem CD137. Signalpeptid (Position +1 bis + 17) und Transmembrandomäne (Position + 187 bis + 213) sind jeweils unterstrichen. Potentielle Glykosylierungsstellen sind mit Sternen gekennzeichnet; Potentielle Phosphorylierungsstellen (Position + 242 für Protein Kinase C; Positionen + 234 und + 235 für Casein Kinase II sind ebenfalls angegeben) das Polyadenylierungssignal ist fettgedruckt dargestellt; (B) ein Alignment der Aminosäurensequenzen von humanem und murinem CD137; identische Aminosäuren sind durch vertikale Linien dargestellt; Aminosäuren mit hoher, geringer oder fehlender Ähnlichkeit sind mit Doppelpunkt, Punkt bzw. Blank gekennzeichnet.
- Fig. 2: die Induktion von Monozyten-Apoptose durch CD137.
- Fig. 3: (A) die Induktion der Proliferation peripherer Monozyten durch immobilisiertes CD137-Fc-Protein anhand des ³H-Thymidineinbaus; (B) die CD137-induzierte Koloniebildung von Monozyten.
- Fig. 4: den Einfluß von M-CSF und GM-CSF auf die CD137-induzierte Monozytenproliferation; (A) Kultivierung peripherer Monozyten auf immobilisiertem Fc- oder CD137-Fc-Protein in Abwesenheit oder Gegenwart neutralisierender Antikörper gegen M-CSF, GM-CSF und/oder IL-3; (B) eine Proliferation von Monozyten wird durch M-CSF und GM-CSF nicht induziert; es ist jeweils die ³H-Thymidin-Aufnahme dargestellt.
- Fig. 5: die Induktion von (A) Wachstum und (B) Proliferation von Monozyten durch konditionierten Überstand von Zellkulturen, welche mit CD137-Fc-Protein kultiviert worden waren.
- Fig. 6: einen Vergleich der Induktion von Monozyten-Proliferation durch lösliches CD137-Fc (sCD137-Pc) und immobilisiertes CD137-Fc; als Kontrolle ist immobilisiertes Fc, lösliches Fc (sFc) sowie der unbehandelte Träger angegeben.
- Fig. 7: Versuche zur Induktion der Monozyten-Proliferation mit Fc, CD137-Fc, TNFR-Fc und anti-CD68.
- Fig. 8: Versuche zur Induktion der Monozyten-Proliferation mit Fc (helle Balken) bzw. CD137-Fc (schwarze Balken) kombiniert mit LPS oder M-CSF.
- Fig. 9: eine fotografische Darstellung von peripheren Monozyten (A) kultiviert über immobilisiertem Fc oder CD137-Fc, 400-fache Vergrößerung; (B) nach 10-tägiger Kultivierung über immobilisiertem CD137-Fc; 500-fache Vergrößerung.
- Fig. 10: in der linken Hälfte der Graphik die Expression von M-CSF durch Monozyten nach 1- bis 8-tägiger Kultivierung über immobilisiertem Fc (weiße Balken) oder immobilisiertem CD137-Fc (schwarze Balken); und in der rechten Hälfte der Grafik die Expression von M-CSF in Gegenwart von löslichem Fc bzw. löslichem CD137-Fc nach 8-tägiger Kultivierung.
- Fig. 11: die Anzahl lebender Monozyten nach Neutralisation von M-CSF; es ist angegeben, die Anzahl lebender Monozyten behandelt mit PC (Kreise) oder CD137-Fc (Quadrate) oder mit 100 ng/ml M-CSF (Dreiecke) ohne (helle Symbole) bzw. nach Zugabe von 2 µg/ml neutralisierendem Anti-M-CSF-Antikörper (schwarze Symbole).
- Fig. 12: Monozyten nach 8-tägiger Kultivierung bei 300-facher Vergrößerung in Gegenwart von löslichem bzw. immobilisiertem Fc oder CD137-Fc-Protein (jeweils 1 µg/ml).

Isolierung, Sequenzierung und Charakterisierung von humanem CD137 wurden erstmals beschrieben von Schwarz, H., et al., in Gene (1993), 134, 295, worauf hiermit ausdrücklich Bezug genommen wird.

Die Erfindung betrifft, wie erwähnt, die Verwendung von CD137 sowie von "funktionaler Analoga" davon. Funktionale Analoga im Sinne der vorliegenden Erfindung sind insbesondere Varianten, Derivate, lösliche Formen und multimere Formen von CD137, welche trotz Abweichung von der nativen Form von CD137 die erfindungsgemäß gewünschte biologische Aktivität besitzen und somit zu den genannten Zwecken einsetzbar sind. Insbesondere müssen die erfindungsgemäßen funktionalen Analoga weiterhin die Fähigkeit besitzen, an die Zielzellen, d.h. an die peripheren Monocyten, zu binden, um auf diesem Weg Monozyten-Proliferation induzieren zu können.

CD137-Varianten umfassen beispielsweise Proteine, welche durch eine oder mehrere Aminosäure-Substitutionen, Deletionen, Additionen, Insertionen und/oder Inversionen, ausgehend von der in Fig. 1A dargestellten Sequenz erhältlich sind.

Erfindungsgemäße Varianten von CD137 sollten eine etwa 60- bis 100-%ige, wie z.B. etwa 80- bis 100-%ige, Übereinstimmung mit der Aminosäuresequenz gemäß Fig. 1A besitzen. Die Veränderungen der nativen Aminosäuresequenz können in an sich bekannter Weise, wie z.B. durch Mutation der entsprechenden Nukleotide in der Nukleotidsequenz hergestellt werden. So können beispielsweise untereinander substituiert werden: Aminosäuren mit ähnlichem aliphatischen Rest, wie Isoleucin, Valin, Leucin und Alanin; Reste mit ähnlicher polarer Seitengruppe, wie Lysin und Arginin; wie Glutamin und Asparagin; oder Glutaminsäure und Asparaginsäure.

Funktionale Analoga umfassen außerdem nicht-glykosilierte oder unterschiedlich glykosilierte Formen von CD137. Das Glykosilierungsmuster kann beispielsweise durch gezielte Wahl des Expressionssystems bei rekombinanter Herstellung von CD137 beeinflußt werden. Außerdem besteht die Möglichkeit, die Aminosäuresequenz im Bereich potentieller N-Glykosilierungsstellen gezielt so zu verändern, dass keine Glykosilierung mehr erfolgt.

Funktionale Analoga umfassen außerdem natürlich vorkommende Varianten von CD137, welche beispielsweise durch alternatives mRNA-Splicing oder durch proteolytische Spaltung von CD137 anfallen und dabei N- oder C-terminal verkürzte Aminosäuresequenzen aufweisen können. Außerdem können Varianten dadurch erhalten werden, daß man gezielt solche terminalen oder internen Aminosäuren oder Aminosäureteilsequenzen deletiert, welche für die gewünschte biologische Funktion nicht von Bedeutung sind. Beispielsweise können auch gezielt Cystein-Reste deletiert oder substituiert werden, um die Ausbildung falscher intermolekularer Disulfidbrücken zu vermeiden.

Derivate von CD137 können einen oder mehrere chemische Reste enthalten, welche durch chemische oder enzymatische Modifikation über funktionale Seitengruppen von Aminosäureresten gebunden sind.

Derivate von CD137 erhält man durch Derivatisierung funktionaler Gruppen an Aminosäureseitenketten oder am N-Terminus oder C-Terminus des Proteins. Beispielsweise sind Glykosylgruppen, Acylgruppen, Lipidreste, Phosphatgruppen, Polymerreste, wie z.B. Polyethylenglykolseitenketten, in an sich bekannter Weise einführbar.

Eine besondere Form der Derivatisierung stellt die N- oder C-terminale Verknüpfung mit einer anderen Aminosäuresequenz dar. Fusionsproteine dieses Typs sind sowohl chemisch als auch in rekombinanter Weise herstellbar.

Erfindungsgemäße Analoga umfassen außerdem lösliche Formen von CD137. Diese umfassen die extrazelluläre Domäne des Proteins ganz oder teilweise, während die Transmembrandomäne teilweise oder ganz deletiert ist. Außerdem weisen diese Formen den cytoplasmatischen, C-terminalen Sequenzteil nicht auf. Lösliche Formen von CD137 sind erfindungsgemäß insbesondere bei in vivo-Anwendungen von Vorteil, da dadurch beispielsweise die intravenöse Verabreichung eines pharmazeutischen Präparates deutlich vereinfacht wird. Eine bevorzugte lösliche Form von CD137 ist ein Polypeptid mit den Aminosäureresten +18 bis +186 gemäß Fig. 1A. Auch andere Analoga in Form von löslichen oder unlöslichen funktionalen Fragmenten, d.h. Teilsequenzen oder zusammengesetzten Teilsequenzen der nativen CD137-Form, sind erfindungsgemäß umfasst.

Die erfindungsgemäßen löslichen Formen von CD137 sind in an sich bekannter Weise herstellbar. Beispielsweise ist eine rekombinante Herstellung ausgehend vom entsprechend verkürzten DNA-Fragment möglich. Außerdem besteht die Möglichkeit, verkürzte CD137-Formen durch gezielten Protease-Verdau herzustellen.

Erfindungsgemäß brauchbare funktionale Analoga zu dem in Fig. 1A konkret beschriebenen Polypeptid umfassen außerdem funktionale äquivalente Polypeptide, wie sie aus anderen Säugern oder anderen zellulären Systemen des gleichen Säugers isolierbar sind. Ein funktionales Äquivalent in diesem Sinne stellt beispielsweise der in der US-Patentschrift 5,674,704 beschriebene, aus Mäusen isolierte Faktor mit der Bezeichnung 4-1BB, sowie die davon wiederum abgeleiteten funktionalen Analoga, wie z.B. Fragmente, dar.

CD137 und dessen oben beschriebene funktionalen Analoga können erfindungsgemäß sowohl in monomeren Form als auch in multimerer Form eingesetzt werden.

Für eine Verwendung in monomerer Form ist eine Immobilisierung des Proteins besonders zweckmäßig. Die Immobilisierung kann dabei in an sich bekannter Weise an einer Trägermatrix erfolgen. Diese Trägermatrix kann z.B. die Oberfläche eines Kulturgefäßes sein, in welchem ein Monozyten-enthaltendes Zellsystem kultiviert werden kann. Eine weitere geeignete Trägermatrix können beispielsweise Polymerpartikel sein, welche in einem Monozyten-enthaltenden Kultursystem suspendierbar sind.

Die optimale Anzahl immobilisierter Moleküle pro Flächeneinheit kann vom Fachmann durch wenige Vorversuche leicht bestimmt werden. Sie kann beispielsweise bei etwa 10¹¹ bis 10¹⁷ CD137-Molekülen oder der funktionalen Analoga davon pro Quadratzentimeter, insbesondere bei etwa 10¹³ bis 10¹⁴, wie z.B. etwa 6*10¹³ liegen.

Die Immobilisierung kann auf jede übliche, dem Fachmann bekannte Art und Weise erfolgen, solange dadurch die gewünschte biologische Aktivität des immobilisierten CD137-Moleküls nicht oder nur unwesentlich beeinträchtigt wird. Die Immobilisierung kann beispielsweise durch Adsorption des Moleküls an einem Träger oder durch kovalente Verknüpfung mit dem Träger, z.B. unter Verwendung von difunktionalen Linker-Molekülen erfolgen, welche z.B. über funktionelle Gruppen von Aminosäureresten gebunden werden. Dabei können z.B. Amid- , Diazo- , Isothiocyanat- oder Disulfidbrücken zwischen dem Linker und CD137 ausgebildet werden (vgl. z.B. Vitetta, et al., 1987, Science 238,1098; Pastan, et al., 1986, Cell, 47, 641; oder Thorpe, et al., 1987, Cancer Res, 47, 5924).

Eine weitere Möglichkeit der Immobilisierung besteht in der Expression von CD137 auf der Zelloberfläche von mit CD137-kodierender DNA transformierten Zellen, wie z.B. CHO-Zellen.

Weiterhin besteht die Möglichkeit, die CD137-Aminosäuresequenz gezielt zu modifizieren, um eine Immobilisierung zu erleichtern. Derartige Modifikationen sind beispielsweise als Anker fungierende sogenannte "Tags", wie z.B. die als Hexa-Histidin-Anker bekannte Modifikation, oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung von CD137 an einen festen Träger, wie z.B. einer Polymermatrix, dienen, der beispielsweise in einer Chromatographiesäule, einer Mikrotiterplatte oder einem Kulturgefäß vorliegen kann.

Eine besonders geeignete Ausführungsform eines zur Immobilisierung geeigneten funktionalen Analogons von CD137 stellt ein Fusionsprotein aus der extrazellulären Domäne von CD137 und dem Fc-Teil eines IgG-Moleküls dar. Derartige Fusionsmoleküle sind in besonders vorteilhafter Weise an Trägern immobilisierbar, an welche entweder Protein A oder ein Anti-Fc-Antikörper gebunden ist. Die Herstellung von CD137-Fc wird in Schwarz, H. et al., Blood, 87, 7 (1996), 2839-2845 beschrieben, worauf hiermit ausdrücklich Bezug genommen wird.

Erfindungsgemäß brauchbare multimere Aggregate von CD137 umfassen vorzugsweise 2 bis 5 CD137-Moleküle oder funktionale Analoga davon. Die Aggregierung der einzelnen Peptidmoleküle hat so zu erfolgen, daß deren Bindung an Monozyten nicht verhindert wird. Vorzugsweise stellt man multimere CD137-Aggregate aus den oben beschriebenen Fusionsmolekülen her, welche die extrazelluläre Domäne von CD137 und den Fc-Teil eines IgG-Moleküls umfassen. Die Dimerisierung kann beispielsweise durch Vernetzung mit einem Anti-Fc-Antikörper erfolgen. Auf diese Weise erhält man ein dimeres Aggregat von CD137. Eine Dimerisierung ist außerdem erreichbar durch Ausbildung von Disulfidbrücken zwischen dem Fc-Teil zweier Fusionsproteinmoleküle. Durch Verwendung von bifunktionellen chemischen Linkern, wie z.B. solchen mit endständigen Sulfydrylgruppen, wie Dithiobis(succinimdyl)propionat, N-Succinimidyl-3(2-pyridyldithio)propionat, oder reaktiven Carbodiimiden, wie z.B. 1-Ethyl-3-(3-dimethylaminopropyl)carbodiimid-Hydrochlorid ist ebenfalls eine Dimerisierung möglich.

Höhere Multimere, wie z.B. Moleküle mit fünf CD137-Einheiten sind erhältlich, ausgehend von dem Fc-Teil pentamerer IgM-Moleküle und der extrazellulären Domäne von CD137.

Ein erster Gegenstand der Erfindung betrifft die Verwendung des Monozytenwachstumsfaktors bezeichneten Zelloberflächenproteins CD137, welches von aktivierten T-Lymphocyten produziert wird, oder eines funktionalen Analogons davon zur Herstellung eines Medikaments zur Förderung der Proliferation peripherer Monozyten, gegebenenfalls auch von Progenitor- und/oder Präkursorzellen (vgl. unten) davon, eines Säugers.

Eine "Förderung" der Monozytenproliferation im Sinne der Erfindung umfaßt sowohl die Induktion der Proliferation nicht-proliferierender Monozyten als auch die zusätzliche Unterstützung bereits proliferierender Monozyten.

Periphere Monozyten sind Bestandteil des peripheren Blutsystems und beim Erwachsenen in einer Konzentration von 80 bis 540 und beim Kind in einer Konzenration von 80 bis 720 Zelle pro µl Blut unter physiologischen Bedingungen enthalten.

Verschiedenste Krankheitszustände sind bekanntlich mit einer Verringerung der Zahl der Monozyten und/oder davon abgeleiteter Zellen, insbesondere Makrophagen unterschiedlicher Gewebespezifität (vgl. oben), assoziiert. Aufgrund der bekannten physiologischen Funktionen von Monozyten und davon abgeleiteter Zellen kann außerdem geschlossen werden, daß eine Erhöhung der Monozytenzahl, z.B. von einem Wert im unteren physiologischen Bereich auf einen darüberliegenden physiologischen Wert, durch vermehrte Proliferation ebenfalls von therapeutischem Nutzen sein könnte. Außerdem ist denkbar, daß durch künstliche Induktion einer Monozytose (Erhöhung der Monozytenzahl im Blut auf Werte von mehr als 540 Zellen pro µl Blut) das monozytäre Abwehrsystem des Körpers bei der Infektabwehr unterstützt wird.

Ein weiterer Gegenstand der vorliegenden Erfindung betrifft deshalb die Verwendung von CD137 oder eines funktionalen Analogons davon zur Herstellung eines Medikaments zur Behandlung einer Erkrankung, die mit einer Störung eines Zellsystems assoziiert ist, das Monozyten und/oder davon abgeleiteten Zellen und/oder Progenitoren und/oder Präkursoren davon, insbesondere Monozyten und/oder davon abgeleitete Zellen, wie Makrophagen, umfasst (vgl. schematische Darstellung des hematopoetischen Systems in Concepts of Gene Therapy, (1997) Verlag Walter de Gruyter Berlin, Strauss, M., und Barranger, J.A. (Hrsg.), S. 236, Tab. 12.1 worauf hiermit ausdücklich Bezug genommen wird); oder deren Entstehung und/oder Verlauf durch Förderung der Proliferation von Zellen dieses Zellsystems therapierbar ist.

Ein Störung in obigem Sinn kann dabei eine angeborene oder erworbene, permanente oder vorübergehende, teilweise oder vollständige Beinträchtigung einzelner oder mehrerer physiologischer Funktionen der betreffenden Körperzellen umfassen.

Ein solches Zellsystem ist beispielsweise das sogenannte myeloische Zellsystem, dessen Zellen von Progenitorzellen aus dem Knochenmark abgeleitet sind. Typische Bestandteile dieses Systems sind Granulozyten und Monozyten.

Insbesondere ist die erfindungsgemäße Verwendung dann indiziert, wenn die genannte Störung eine funktionelle Störung oder eine Verringerung der Monozytenzahl unter eine Konzentration von 80 Zellen je µl Blut, und/oder eine Veringerung davon abgeleiteter Zellen umfasst.

Ein erstes bevorzugtes Anwendungsgebiet für CD137 stellen Erkrankungen dar, die ausgewählt sind unter Chemotherapie- oder Strahlentherapie-bedingten Schädigungen des hämatopoetischen Systems. Chemo- und Strahlentherapie werden häufig zur Behandlung verschiedenster Tumorerkrankungen oder im Rahmen einer myelosuppressiven oder myeloablativen Therapie zur Vorbereitung von Knochenmarks- oder Organtransplantationen durchgeführt. Leukopenie und eine damit verbundene Verringerung der Monozytenzahl ist dabei häufig Folge. Morbidität und Mortalität der Patienten aufgrund erhöhter Infektionsanfälligkeit nehmen stark zu. Durch erfindungsgemäße Verabreichung von CD137 oder CD137-behandelten Monozyten könnte die Leukopenie wirksamer lindern bzw. beseitigen.

Ein weiteres Anwendungsgebiet betrifft die Verwendung von CD137 oder funktionaler Analoga zur Behandlung von Wundheilungsstörungen, wie sie z.B. bei Dialysepatienten oder Diabetikern oder Patienten mit chronischer venöser Insuffizienz zu beobachten ist. Hierbei handelt es sich um Wundheilungsstörungen, die aus unzureichend vorhandenem oder funktionierendem Granulationsgewebe, welches überwiegend aus Makrophagen (d.h. differenzierten Monozyten) besteht, resultieren.

Ein weiterer Gegenstand der Erfindung betrifft die Verwendung von CD137 oder eines funktionalen Analogons davon zur Herstellung eines Medikaments zur Behandlung von Erkrankungen, die mit einer unzureichenden Immunantwort assoziiert sind. Als Beispiele für derartige Erkrankungen sind zu nennen:
a) Tumorerkrankungen, die durch unzureichende oder fehlende zytotoxische Aktivität des körpereigenen Abwehrsystems begünstigt werden.
b) Bakterielle, virale und Pilzinfektionen, die durch eine unzureichende oder fehlende Phagozytose des Pathogens oder damit infizierter Körperzellen begünstigt werden.

Weiterhin wird eine Behandlung von
c) angeborenen oder nicht-angeborenen, erblichen oder nichterblichen Schädigungen oder Erkrankungen des Immunsystems; und
d) durch Behandlung mit Immunsuppressiva induzierten Schädigungen, wie sie z.B. bei Behandlung von Patienten mit chronischer Polyarthritis oder Autoimmunerkrankungen oder von Transplantationspatienten auftreten können,
ermöglicht.

Wie in einem späteren Abschnitt noch genauer erläutert werden wird, kann das erfindungsgemäße Medikament im Rahmen einer in vivo- oder ex vivo-Behandlung eingesetzt werden.

Gemäß einer weiteren Ausführungsform der Erfindung kann CD137 oder ein funktionales Analogon davon auch in Kombination mit wenigsten einem weiteren Faktor Anwendung finden, der ausgewählt ist unter Interleukinen, Lymphokinen, Monokinen, Interferonen, koloniestimulierenden Faktoren und Wachstumsfaktoren. Als nichtlimitierende Beispiele sind zu nennen: IL-1, IL-2, IL-3, IL-4, IL-5, IL-6, IL-7, IL-8, IL-9, IL-10, IL-11, IL-12, IFN-α, -β, -γ, TNF-α, EGF, TGF, PDGF, ILGF, MGF, EPO, G-CSF, GM-CSF und M-CSF, wobei Leukozyten-stimulierende Faktoren, wie G-CSF, GM-CSP und vor allem M-CSF bevorzugt sind. CD137 und der weitere Faktor können dabei gleichzeitig oder zeitlich versetzt in beliebiger Reihenfolge verabreicht werden.

Denkbar ist eine kombinierte Verwendung mit Apoptose-hemmenden Faktoren. Möglicherweise besitzt M-CSF einer derartigen inhibierenden Effekt auf die Monozyten-Apopotose.

Zur Verwendung im Rahmen der vorliegenden Ertindung eignet sich humanes CD137 grundsätzlich in seiner nativen Form, d.h. als Protein mit einer Aminosäuresequenz von Rest +18 bis Rest +255 in der Sequenz gemäß Fig. 1A oder ein funktionales Analogon dieser Sequenz.

Bevorzugt verwendet man jedoch die extrazelluläre Domäne von humanem CD137 entsprechend den Aminosäuren + 18 bis + 186 gemäß Figur 1A oder ein funktionales Analogon davon.

In einer bevorzugten Ausführungsform der Erfindung wird CD137 oder dessen funktionales Analogon in immobilisierter Form oder als multimeres Aggregat eingesetzt wird.

Ein bevorzugtes multimeres CD137-Aggregat umfasst 2 bis 5 CD137-Proteinmoleküle oder funktionale Analoga davon.

Gegenstand der Erfindung ist außerdem ein in vitro oder ex vivo-Verfahren zur Förderung der Proliferation peripherer Monozyten, wobei man periphere Monozyten aus dem Blut eines Säugers in einem Nährmedium mit freiem oder immobilisiertem, insbesondere immobilisiertem CD137 in Kontakt bringt und inkubiert, bis die Monozytenzahl, vorzugsweise auf ihren Maximalwert, gestiegen ist, und/oder eine Vergrößerung der Monozyten beobachtet werden kann.

Die Isolierung von Monozyten aus dem Blut eines Säugers kann nach allgemein üblichen Standardmethoden erfolgen. Die isolierte Zellfraktion kann entweder eine reine Monozytenfraktion sein oder Zellen enthalten, welche die Behandlung der Monozyten und die nachfolgende Behandlung des Säugers mit den stimulierten Monozyten nicht negativ beeinflussen. So können beispielsweise Lymphozyten während der in vitro-Inkubationsphase neben den Monozyten vorliegen. Geeignete Methoden zur Isolierung von Monozyten sind beispielsweise beschrieben in Meyskens, F.L., et al, Exp. Hematol. 1979, 7(8), 401-410; Weiner, R.S., et al., J. Immunol. Methods, 1980, 36(2), 89-97; und Contreras, T.J., et al., Cell. Immunol., 1980, 54(1), 215-229.

Ein nichtlimitierendes Beispiel für ein zur in vivo oder ex vivo Kultivierung geeignetes Nährmedium ist beispielsweise RPMI-Medium supplementiert mit 5% fetalem Kälberserum. Nach Beendigung der Kultivierung ist es zweckmäßig die behandelte Monozytenfraktion zu waschen, wie z.B. mit PBS, bevor man sie dem zu behandelnden Säuer wieder verabreicht.

Ein weiteres Anwendungsgebiet für CD137 oder eines funktionalen Analogons ist ein Verfahren zur regenerativen Behandlung von Chemotherapie- oder Strahlentherapie-Patienten, wobei man
a) aus dem Blut des Patienten vor Durchführung der Chemooder Strahlentherapie eine periphere Monozyten enthaltende Blutfraktion isoliert, diese ex vivo mit freiem oder immobilisiertem, insbesondere immobilisiertem CD137 inkubiert, bis die Monozytenzahl steigt, bzw. ihr Optimum erreicht hat, und/oder eine Größenzunahme der Monozyten zu beobachten ist, und die auf diese Weise behandelte und vorzugsweise mit Monozyten angereicherte Blutfraktion dem Patienten nach Beendigung der Therapie wieder verabreicht; oder
b) dem Patienten vor, während oder nach Beendigung der Chemooder Strahlentherapie eine wirksame Menge eines multimeren CD137-Aggregats zur Förderung der Proliferation der körpereigenen peripheren Monozyten verabreicht.

Ein weiteres Anwendungsgebiet betrifft die Verwendung von CD137 oder eines funktionalen Analogons in einem Verfahren zur Förderung der körpereigenen unspezifischen Immunabwehr, wobei man einem Patienten eine die Proliferation peripherer Monozyten fördernde Menge von CD137 oder einem funktionalen Analogon davon, insbesondere eine die Proliferation peripherer Monozyten fördernde Menge eines multimeren CD137-Aggregats, verabreicht. Dieses Verfahren eignet sich insbesondere zur Durchführung an Tumorpatienten sowie Patient die an einer bakteriellen, viralen oder Pilzinfektion leiden.

Ein im Rahmen dieses Verfahrens vorteilhaft verabreichbares multimeres CD137-Aggregat umfasst 2 bis 5 CD137-Proteinmoleküle oder funktionale Analoga davon.

Besonders geeignet ist die Verabreichung solcher Analoga oder Aggregate, welche den extrazellulären Abschnitt von humanem CD137 entsprechend den Aminosäuren +18 bis +186 gemäß Figur 1A unfassen oder eines zur Bindung an Monozyten befähigtes funktionales Analogon davon.

Für in vivo-Applikationen ist von Vorteil, humanes CD137 oder ein davon abgeleitetes Analogon zu verwenden. Wird CD137 als Fusionsprotein verabreicht, so sollte das mit CD137 fusionierte Protein ebenfalls humanen Ursprungs sein. Weitere Maßnahmen zur Verbesserung der Verweilzeit von CD137 im Blut, wie z.B. durch PEGylierung, wie sie z.B. für andere Cytokine bereits erfolgreich verwendet wurde (vgl. PEGylierung von G-CSF, beschrieben in EP-A-0 335 423), sind ebenfalls denkbar.

Die jeweilige Wahl der Dosierung von CD137 oder eines funktionalen Analogon davon und der jeweilige Dosierungsplan obliegen der Entscheidung des behandelnden Arztes. Dieser wird in Abhängigkeit vom gewählten Verabreichungsweg, von der Wirksamkeit des jeweiligen Medikaments, von der Art und Schwere der zu behandelnden Erkrankung, von dem Befinden des Patienten und dessen Ansprechen auf die Therapie einen geeignete Dosis und einen entsprechenden Dosierungsplan auswählen. Eine geeignete Dosis sollte aber generell im Bereich von etwa 1 bis 20 µg/kg Körpergewicht/Tag oder im Bereich von etwa 0,01 bis 1 nMol/kg Körpergewicht/Tag liegen.

Bezogen auf Blutvolumen können wirksame Konzentrationen von CD137 oder eines funktionalen Analogon davon während der Therapie im Bereich von etwa 0,01 µg bis 10 µg pro Milliliter Blut oder im Bereich von etwa 0,1 pMol bis 0,5 nMol pro Milliliter Blut liegen.

Für in vitro oder ex vivo Anwendungen können wirksame Konzentrationen von CD137 oder eines funktionalen Analogon davon im Bereich von mehr als etwa 0,1 µg/ml Medium, insbesondere mehr als etwa 1 µg/ml Medium, wie z.B. bis zu etwa 50 µg/ml Medium, oder im Bereich von mehr als etwa 1 pMol/ml Medium, insbesondere mehr als etwa 0,01 nMol/ml Medium, wie z.B. etwa 2 nMol/ml Medium, liegen.

Die in vivo-Applikation von CD137 und der funktionalen Analoga davon erfolgt vorteilhafterweise unter Verwendung eines parenteral, insbesondere intravenös verabreichbaren, flüssigen pharmazeutischen Mittels. Dieses enthält vorzugsweise in einem für diesen Zweck geeigneten, pharmazeutisch akzeptablen Träger, eine wirksame Menge von CD137 oder eines funktionalen Analogon davon, vorzugsweise in gelöster Form. Beispiele für geeignete pharmazeutische Träger sind insbesondere wäßrige Lösungen, wie z.B. physiologische Kochsalzlösung, Phosphat-gepufferte Kochsalzlösung, Ringerlösung, laktierte Ringerlösung und dergleichen. Außerdem kann das Mittel weitere Zusätze, wie Antioxidantien, chelatbildende Mittel oder antimikrobielle Mittel, enthalten. Orale Verabreichung sowie Verabreichung durch Inhalation sind ebenfalls möglich.

Eine intravenöse Verabreichung ist besonders zweckmäßig, wenn eine systemische Therapie erforderlich ist. Zur Behandlung lokaler Erkrankungen, wie z.B. lokal begrenzter Infektionen, kann auch eine gezielte subkutane oder intradermale Verabreichung von Vorteil sein. Zur lokalen Wundbehandlung ist beispielsweise die oberflächliche, kutane Verabreichung, möglich. Diese kann druch Auftragung einer Lösung, einer Suspension, einer Salbe oder eines Gels erfolgen.

Die optimale Konzentration von CD137 oder eines funktionalen Analogon davon in den erfindungsgemäßen pharmazeutischen Mitteln wird unter anderem bestimmt von der spezifischen Aktivität der verwendeten Form von CD137. Geeignete Gewichtsanteile von CD137 oder eines funktionalen Analogon davon sollten aber im Bereich von etwa 0,0001 bis 1 Gew.-%, insbesondere 0,0005 bis 0,01 Gew.-%, bezogen auf das Gesamtgewicht des Mittels liegen. Die molare Konzentration kann beispielsweise im Bereich von etwa 1 nMol bis 0,1 mMol, insbesondere etwa 15 bis 300 nMol pro 100g des eingesetzten Mittels liegen.

Gegenstand der Erfindung ist außerdem die Verwendung einer Nukleotidsequenz, welche für CD137 oder ein funktionales Analogon davon kodiert, zur Herstellung eines gentherapeutischen Mittels zur Behandlung einer der in oben definierten Erkrankungen.

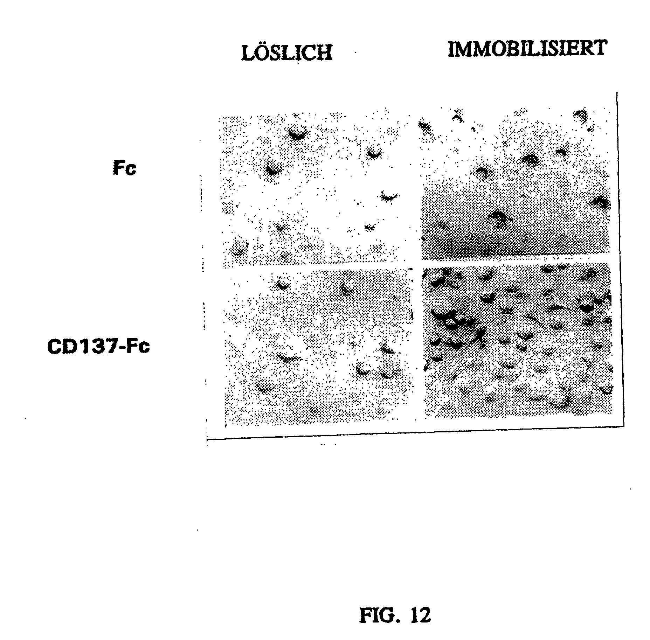

Derartige gentherapeutisches Mittel, umfassend einen zellulären Träger, insbesondere periphere Monozyten, davon abgeleitete Zellen oder Progenitorzellen oder Präkursoren der Monozyten (vgl. schematische Darstellung des hematopoetischen Systems in in Concepts of Gene Therapy, (1997) Verlag Walter de Gruyter Berlin, Strauss, M., und Barranger, J.A. (Hrsg.) S. 236, Fig. 12.1; worauf hiermit ausdrücklich Bezug genommen wird), in welchen die kodierende Nukleotidsequenz für CD137 oder für ein funktionales Analogon davon in exprimierbarer Form in ein geeinetes Nukleinsäurekonstrukt eingebracht ist.

Der Gentransfer in die genannten Zellen kann dazu in an sich bekannter Weise erfolgen, wie z.B. mit Hilfe von viraler Konstrukte, nichtvirale Vehikel, wie Liposomen oder sonstiger geeigneter Nukleinsäurekonstrukte (Günzburg, W.H. et al., Gentransfer in Säugerzellen, (1997) Spektrum Akademischer Verlag, Heidelberg, Berlin; Baum, C., et al Gen Transfer and Transgene Expression in Hematopoietic Cells, in Concepts of Gene Therapy, (1997) Verlag Walter de Gruyter Berlin, Strauss, M., und Barranger, J.A. (Hrsg.) 233-256).

Das erfindungsgemäß verwendbare Nukleinsäurekonstrukt wird beispielsweise mit einem Virusvektor kombiniert, wie z.B. mit einem Adenovirusvektor oder einem replikationsdefizienten Adenovirusvektor, oder mit einem Adeno-assoziierten Virusvektor ligiert.

Eine weitere vorteilhafte Kombination ist die Komplexierung der Nukleinsäurekonstrukte mit Liposomen. Bei der Lipofektion werden kleine unilamellare Vesikel aus kationischen Lipiden durch Ultraschallbehandlung der Liposomensuspension hergestellt. Die DNA wird ionisch auf der Oberfläche der Liposomen gebunden und zwar in einem solchen Verhältnis, daß eine positive Nettoladung verbleibt und die DNA zu 100% von den Liposomen komplexiert wird. Neben Lipidmischungen aus DOTMA (1,2-Dioleyloxypropyl-3-trimethyl-ammoniumbromid) und DOPE (Dioleoylphosphatidylethanolamin) wurden inzwischen zahlreiche neue Lipidformulierungen synthetisiert und auf ihre Effizienz der Transfektion verschiedener Zellinien getestet (Behr, J. P. et al. (1989) Proc. Natl. Acad. Sci USA 86, 6982-6986; Felgner, J. H. et al. (1994) J. Biol. Chem. 269, 2550-2561; Gao, X. & Huang, L. (1991) Biochem. Biophys. Res. Commun. 179, 280-285; Zhou, X. & Huang, L. (1994) Biochim. Biophys. Acta 1189, 195-203). Beispiele der neuen Lipidformulierungen sind DOTAP N-[1-(2,3-Dioleoyloxy)propyl]-N,N,N-trimethylammoniummethylsulfat oder DOGS (TRANSFECTAM; Dioctadecylamidoglycylspermin).

Neben der für CD137 oder einem funktionalen Analogon davon kodierenden Nukleotidsequenz umfassen brauchbare Nukleinsäurekonstrukte in funktioneller, operativer Verknüpfung eine oder mehrere regulative Sequenzen, wie Promotoren, Amplifikationssignale, Enhancer, Polyadenylierungssequenzen, Replikationsursprünge, Reportergene, selektierbare Markergene und dergleichen. Diese Verknüpfung kann je nach gewünschter Anwendung zu einer Erhöhung oder Erniedrigung der Genexpression führen.

Zusätzlich zu den neueingeführten Regulationssequenzen kann die natürliche Regulationssequenz vor den eigentlichen Strukturgenen noch vorhanden sein. Durch genetische Veränderung kann diese natürliche Regulation gegebenenfalls ausgeschaltet und die Expression der Gene erhöht werden. Das Genkonstrukt kann aber auch einfacher aufgebaut sein, das heißt es werden keine zusätzlichen Regulationssignale vor die Strukturgene insertiert und der natürliche Promotor mit seiner Regulation wird nicht entfernt. Stattdessen wird die natürliche Regulationssequenz so mutiert, daß keine Regulation mehr erfolgt und die Genexpression gesteigert wird. Auch am 3'-Ende der Nukleinsäuresequenzen können zusätzliche vorteilhafte regulatorische Elemente insertiert werden. Die Nukleinsäuresequenzen können in einer oder mehreren Kopien im Genkonstrukt enthalten sein.

Prinzipiell können alle natürlichen Promotoren mit ihren Regulationssequenzen verwendet werden. Darüber hinaus können auch synthetische Promotoren vorteilhaft verwendet werden. Die regulatorischen Sequenzen sollen vorzugsweise die gezielte Expression der Nukleinsäuresequenzen ermöglichen.

Die vorliegende Erfindung wird nun anhand folgender nicht-limitierender Ausführungsbeispiele näher erläutert.

### Ausführungsbeispiele

### Reagenzien

M-CSF sowie neutralisierende Antikörper gegen M-CSF, GM-CSF und IL3 wurden von R&D (Wiesbaden, Deutschland) bezogen. Anti-M-CSF: Klon 26730,11, Protein-A-gereinigte IgG-Fraktion aus der Ascitesflüssigkeit von Maushybridom. Anti-GM-CSF: Klon 3209.1, monoklonaler IgG₁ Maus-Antikörper; Anti-IL3: Protein-A-gereinigte IgG-Fraktion aus der Ascitesflüssigkeit von Maushybridom. Rekombinantes CD137-Fc-Protein, bestehend aus der extrazellulären Domäne von humanem CD137 und der konstanten Domäne von menschlichem Immunglobulin G₁ (Fc) wurde von Alexis (Grünberg, Deutschland) bezogen.

Humanes IgG₁ Fc-Protein wurde von der Accurate Chemical and Scientific Corporation (Westbury, NY, USA) bezogen.

### Referenzbeispiel 1: Immobilisierung von CD137-Fc

Polystyrol-Mikrotiterplatten (Microtest III Tissue Culture Plates; Becton Dickinson, Franklin Lakes NJ, USA) wurden mit einer Lösung von 1 µg/ml CD137-Fc-Protein in PBS (Phosphat-gepufferte Kochsalzlösung) über Nacht bei 4°C inkubiert. Je Vertiefung wurden 50 µl der Lösung verwendet. Am nächsten Morgen wurde die Proteinlösung abgehoben und die Platten wurden mit PBS gewaschen. Die Immobilisierung von Fc erfolgte in analoger Weise.

### Referenzbeispiel 2: ELISA

ELISA-Kits wurden von R&D Systems (Wiesbaden, Deutschland) bezogen. Die Durchführung erfolgte gemäß den Angaben des Herstellers. Cytokinkonzentrationen wurden mit dem Test 3-fach bestimmt und als Mittelwert ± Standardabweichung ausgedrückt.

### Referenzbeispiel 3: Bestimmung der Apoptose von Monozyten

Die DNA-Fragmentierung wurde mit Hilfe des "Cell Death Detection ELISA" (Boehringer Mannheim, Deutschland) gemäß den Anweisungen des Herstellers bestimmt. Die Messungen wurden 3-fach durchgeführt.

### Referenzbeispiel 4: Isolierung und Kultivierung von humaner Monozyten

Humane Peripherblut-Mononukleare Zellen (PBMC) wurden aus Buffy coats gesunder Probanden isoliert. Buffy coats wurden dazu mit zwei gleichen Volumina PBS verdünnt. Damit wurde ein gleiches Volumen Histopaque (Sigma, Deisenhofen, Deutschland) überschichtet. Dieser Ansatz wurde 20 min bei 1200 g zentrifugiert. PBMC's, die sich in Form einer weißen Schicht an der Percoll-Trennfläche anreicherten, wurden isoliert. Erythrozyten wurden mit 2 ml 200 mM NH₄Cl, 10 mM NaHCO₃, 10 mM EDTA, pH 7,4 für 2 min bei Raumtemperatur lysiert. Die Zellen wurden zweimal mit PBS gewaschen, bei 250 g pelletiert und in RPMI-Medium, supplementiert mit 5% fötalem Kälberserum, resuspendiert.

Die primären Monozyten wurden daraus durch Elutriation (Andreesen, R., et al, J Leukoc Biol 47:490, 1990) isoliert. Die elutriierten Monozyten waren zu 95% rein und der Gehalt an T-Lymphozyten betrug weniger als 3% (abgeschätzt anhand der Morphologie sowie des Antigenphenotyps, d.h. Expression von DC14, DC3, CD4 und CD8). Die Zellen wurden in Polystyrol-Kulturschalen (Becton Dickinson, Franklin Lakes, NJ, USA) in RPMI 1640-Medium, supplementiert mit 5% FCS, bei derjeweils angegebenen Zellkonzentration kultiviert.

### Referenzbeispiel 5: Bestimmung der Zellproliferation

Zur Messung der Proliferation einzelner Zellen wurde der "In Situ Proliferation Kit" von Boehringer Mannheim, Deutschland, verwendet. Je Kammer eines 8-kämmrigen Trägers (FALCON, Becton Dickinson, Heidelberg, Deutschland), welche mit Fc oder CD137-Fc-Protein beschichtet worden waren, wurden 3 x 10⁵-Zellen angeimpft und 10 Tage kultiviert. 10 µM Bromdesoxyuridin (BrdU) wurde 60 min zugesetzt. Inkorporiertes BrdU wurde entsprechend der Testanleitung durch Anfärben mit Maus-Anti-BrdU sowie Schaf-Anti-Maus-FITC visualisiert. Monozyten wurden durch Anfärben mit Phycoerythrin-markiertem Anti-CD14-Antikörper (2 µg/ml; Immunotech, Marseille, Frankreich) identifiziert. Chromatin wurde mit 5 µg/ml Hoechst 33342 (Sigma, Deisenhofen, Deutschland) 5 min angefärbt.

Die Proliferation von Zellpopulationen wurde in einer 96-Loch-Mikrotiterplatte bestimmt. 10⁵ Monozyten je Loch wurden mit 0,5 µCi ³H-Thymidin 24 h gepulst, geerntet und mit einem TopCount Mikroplatten-Scintillationszähler (Packard, Meriden, CT, USA) gezählt. Jeder Ansatz wurde dreimal durchgeführt und die Ergebnisse sind als Mittelwerte ± Standardabweichung angegeben.

### Beispiel 1: CD137-Fc induziert Apoptose in Monozyten

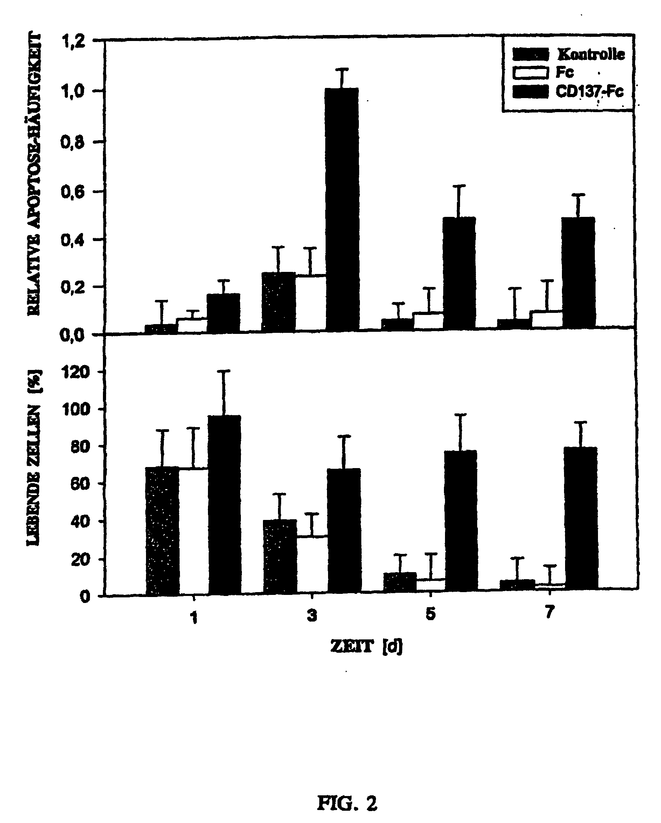

10⁵ primäre Monozyten wurden auf Gewebekultur-Schalen kultiviert, die zuvor mit einem Fusionsprotein beschichtet worden waren, welches aus der extrazellulären Domäne von CD137 und der konstanten Domäne von menschlichem Immunglobulin G₁ (Fc) bestand. Unbehandelte und mit Fc-Protein beschichtete Platten wurden als Kontrollen verwendet. In den mit CD137-Fc-Protein beschichteten Vertiefungen war die Anzahl lebender Monozyten an den Tagen 1, 3, 5 und 7 der Kultivierung signifikant erhöht (Fig. 2, untere Hälfte). Der Grad der Apoptose in den gleichen Kulturen wurde über die Menge fragmentierter DNA bestimmt (Fig. 2, obere Hälfte). Überraschenderweise wurde ein höherer Apoptose-Grad in Monozytenkulturen festgestellt, welche mit CD137-Fc-Protein behandelt worden waren. Vergleichbare Resultate erhielt man in drei unabhängigen Experimenten.

### Beispiel 2: CD137-Fc induziert die Proliferation von peripheren Monozyten und bewirkt eine Koloniebildung der Monoyten

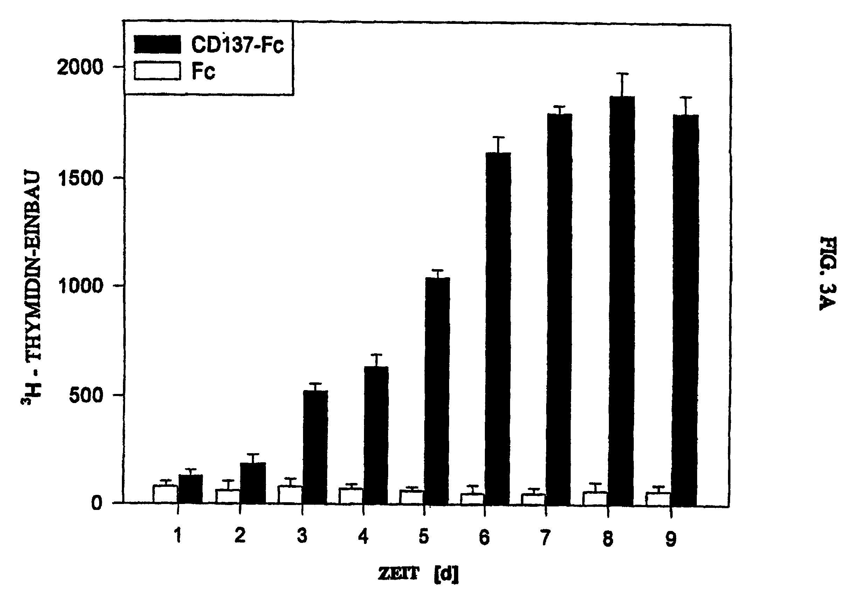

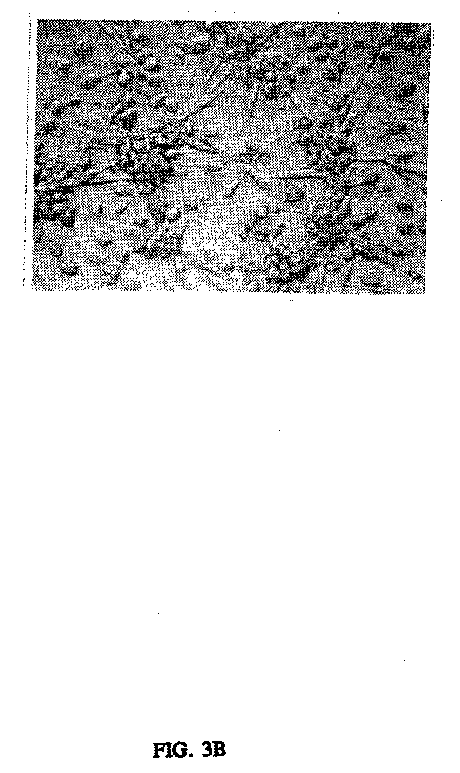

(a) CD137 induzierte eine starke Proliferation von Monozyten, welche den Verlust an Zellen durch ebenfalls induzierte Apoptose überkompensierte. 10⁵-Monozyten wurden in 96-Loch-Platten, beschichtet mit Fc oder CD137-Fc, kultiviert. Die Proliferation wurde täglich durch einen 24-Stunden-Puls mit 0,5 µCi ³H-Thymidin bestimmt. Wie die ³H-Thymidineinbauraten zeigten, induzierte CD137-Fc in der Tat eine starke Monozytenproliferation (Fig. 3A). Die Proliferation zeigte eine positive Korrelation mit der Kultivierungsdauer der Monozyten in Gegenwart von CD137-Fc-Protein. Die Induktion der Proliferation erreichte ein Maximum nach 7 bis 10 Tagen, wobei der ³H-Thymidin-Einbau im Vergleich zu Kontrollzellen 30-fach oder mehr erhöht war. Es wurde kein Unterschied zwischen den Monozyten in unbehandelten und mit Fc-Protein beschichteten Vertiefungen festgestellt (Ergebnisse nicht gezeigt).
   Die substantielle Zunahme der ³H-Thymidinaufnahme steht in Übereinstimmung mit dem Befund, das die CD137-Fc-induzierte Proliferation weit verteilt in der Kultur erfolgte. Bei Markierung der CD137-behandelten Monozyten durch einstündige Behandlung mit Bromdesoxyuridin (BrdU) und anschließender Detektion mit einem Fluorescein-markierten Anti-BrdU-Antikörper konnte nämlich festgestellt werden, daß 9,3% der Zellen (55±6 von 589±43) ihre DNA replizierten, während periphere Monozyten, die auf Fc-beschichteten Trägern gezüchtet wurden, keinen Einbau von BrdU zeigten (Ergebnisse nicht gezeigt).
   Durch immunocytochemische Untersuchungen konnte außerdem bestätigt werden, daß die proliferierenden Zellen in der Tat Monozyten und keine andere Blutzellen waren. Die Proliferation wurde nämlich, wie oben beschrieben, durch BrdU-Inkorporation bestimmt und die Identität der Monozyten wurde durch simultane Anfärbung von CD14, einem für Monozyten spezifischen Zelloberflächenprotein, verifiziert. Die Kerne wurden durch Anfärben mit dem DNA-Intercalations-Farbstoff Hoechst 33342 sichtbar gemacht. Bei diesen Untersuchungen wurde festgestellt, daß die proliferierenden Zellen typische Merkmale für Monozyten aufwiesen: Sie waren CD14-positiv, mehrkernig und zeigten die typische Morphologie von Monozyten/Makrophagen (Ergebnisse nicht gezeigt).
(b) Wie die mikroskopische Aufnahme gemäß Fig. 3B zeigt, führt eine erfindungsgemäße Behandlung von Monozyten mit immobilisiertem CD137-Fc zu einer signifikanten Koloniebildung, ähnlich wie es auch für andere hämatopoetische Wachstumsfaktoren bereits gezeigt worden ist.

### Beispiel 3: M-CSF und GM-CSF sind wesentliche Zusatzfaktoren für die CD137-induzierte Monozytenproliferation

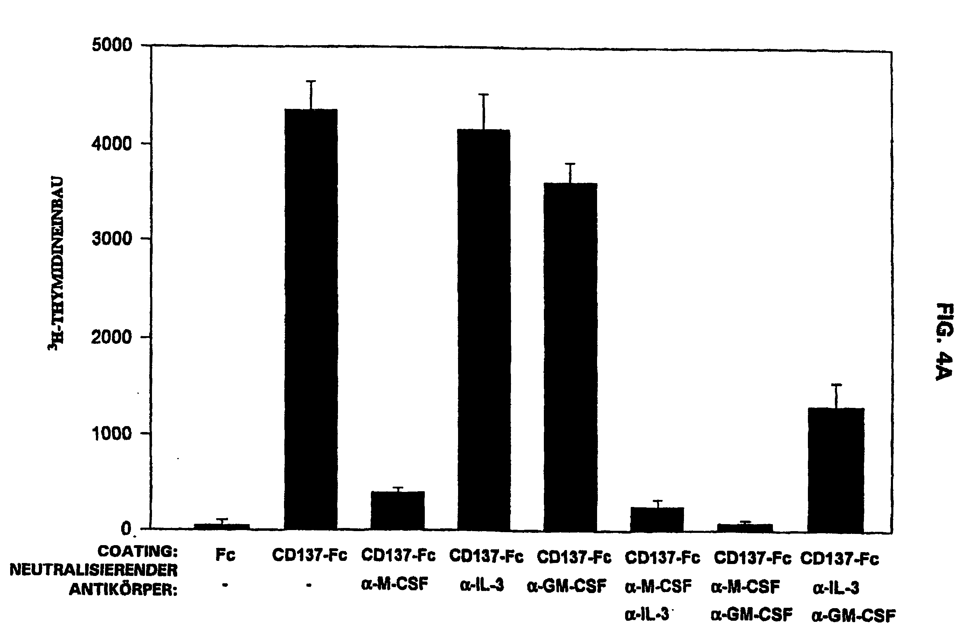

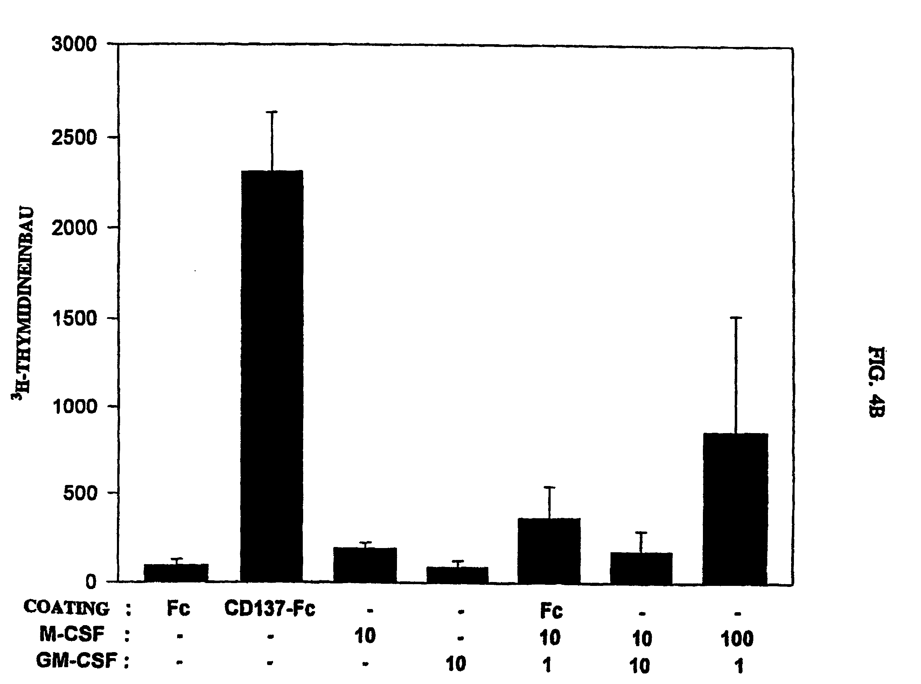

(a) 10⁵ periphere Monozyten wurden über immobilisiertem Fc oder immobilisiertem CD137-Fc kultiviert. Neutralisierende Anti-M-CSF-Antikörper (2 µg/ml), Anti-GM-CSF-Antikörper (2 µg/ml) und Anti-IL-3-Antikörper (2 µg/ml) wurden gemäß Versuchsplan zugesetzt. Die Proliferation wurde am Tag 10 über den 3H-Thymidineinbau bestimmt.
   Durch neutralisierende Anti-M-CSF- und Anti-GM-CSF-Antikörper konnte eine vollständige Inhibition der Monozytenproliferation erreicht werden. Neutralisierender Anti-GM-CSF-Antikörper alleine bewirkte lediglich eine 18%-ige Verringerung der Proliferation. Anti-IL-3-Antikörper hatte keinen Einfluß auf die CD137-induzierte Proliferation. Die Zugabe von Anti-GM-CSF- und Anti-IL-3-Antikörper führte zu einer synergistischen Verringerung der Proliferation auf ein Drittel des ursprünglichen Werts (vgl. Fig. 4A).
   Diese Ergebnisse veranschaulichen, daß M-CSF und GM-CSF wesentliche Faktoren für die CD137-induzierte Proliferation von peripheren Monozyten darstellten.
(b) In einem weiteren Versuch wurde jedoch festgestellt, daß M-CSF und GM-CSF bei Konzentrationen, wie sie durch CD137 induziert werden, lediglich eine vernachlässigbare Proliferation hervorriefen. Periphere Monozyten wurden auf 96-Loch-Platten, beschichtet mit Fc oder CD137-Fc-Protein (1 µg/ml) oder mit M-CSF und GM-CSF bei den angegebenen Konzentrationen (ng/ml) kultiviert. Die Proliferation wurde am Tage 10 über den ³H-Thymidineinbau bestimmt (Fig. 4B). In Vorversuchen wurde festgestellt, daß M-CSF durch CD137 in Konzentrationen von bis zu 10 ng/ml induziert wurde. Eine GM-CSF-Induktion konnte nicht gemessen werden (Ergebnisse nicht gezeigt). In vivo sind M-CSF und GM-CSF in Konzentrationen von 10 ng/ml bzw. 50 pg/ml nachweisbar (Kawano, Y., Eur J Haematol 54:147, 1995; Einer, S.G., Curr Eye Res 14:1045, 1995; Saunders, M.A., Br J Pharmacol 120:545, 1997). In der in Fig. 4B dargestellten Testreihe wurden M-CSF und GM-CSF in im Vergleich zu den Literaturwerten signifikant höheren Konzentrationen, nämlich 100 bzw. 1 ng/ml eingesetzt. Im Vergleich zu CD137 konnte jedoch nur ein Bruchteil der Proliferation durch M-CSF und/oder GM-CSF induziert werden. Dies kann als Hinweis darauf gedeutet werden, daß zusätzliche Faktoren, die über die Einwirkung von CD137 in Monozyten induziert werden, autokrin zur CD137-induzierten Monozytenproliferation beitragen.

### Beispiel 4: Die CD137-Fc-induzierte Monozytenproliferation wird durch einen oder mehrere lösliche autokrine Faktoren vermittelt

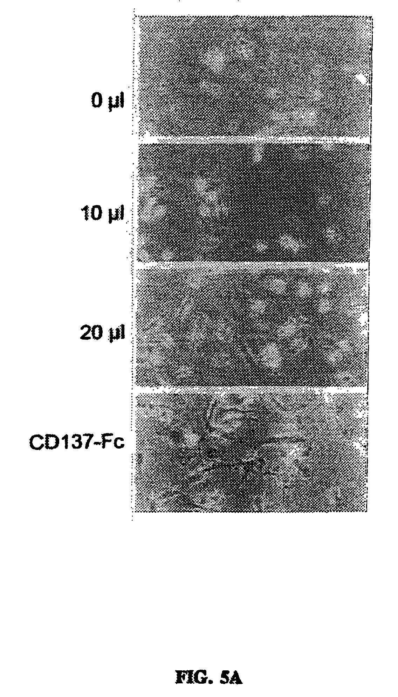

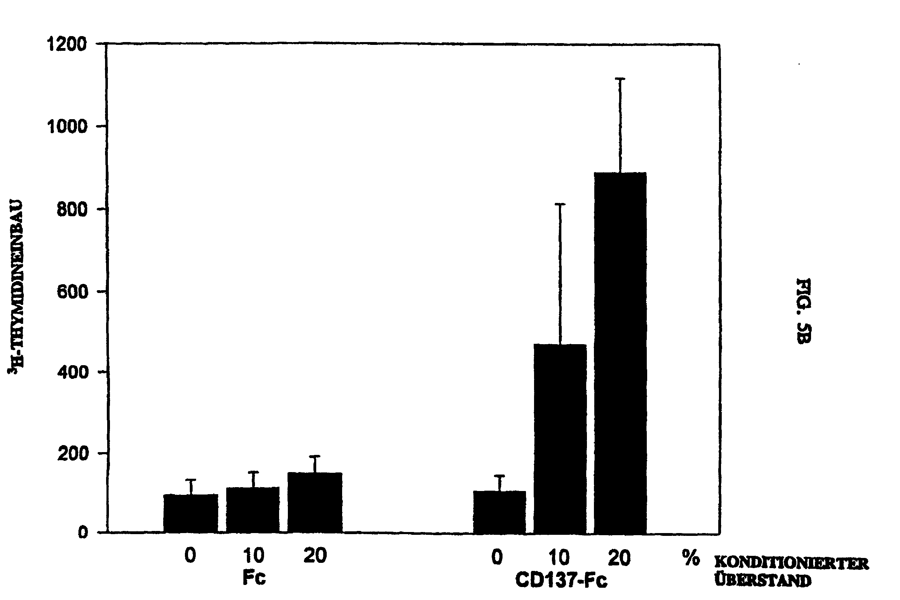

Als nächstes wurde untersucht, ob diese an der CD137-induzierten Monozytenproliferation möglicherweise zusätzlich beteiligten Faktoren in löslicher Form oder gebunden an der Zelloberfläche vorliegen. Dazu wurden 10⁵ periphere Monozyten auf immobilisiertem Fc oder immobilisiertem CD137-Fc-Protein 24 h kultiviert. Die Zellen wurden durch 5-minütige Zentrifugation bei 12000 g entfernt. 0, 10 und 20 µl konditionierter Überstand dieser Kulturen wurde auf 100 µl frischer Kulturen mit unbehandelten Monozyten übertagen. Die Übertragung dieses konditionierten Mediums auf unbehandelte Monozyten des gleichen Donors induzierte in Dosis-abhängiger Weise Zellwachstum (Fig. 5A) sowie Zellproliferation (Fig. 5B). Zur Veranschaulichung des Einflusses auf das Zellwachstum wurden die Zellen nach 8 Tagen bei 300-facher Vergrößerung fotografiert. Die untere Abbildung in Fig. 5A zeigt zum Vergleich Monozyten, die 8 Tage auf immobilisiertem CD137-Fc-Protein kultiviert worden waren.

Zur Bestimmung der Proliferation (Fig. 5B) wurde Fc bzw. CD137-Fc in der obenbeschriebenen Weise auf Kulturträgern immobilisiert. Anschließend wurden Monozyten mit konditioniertem Überstand in der angegebenen Konzentration kultiviert. Die Proliferation wurde, wie oben beschrieben, über den ³H-Thymidineinbau bestimmt. Dazu wurde am Tag 8 24 Stunden mit 0,5 µCi ³H-Thymidin gepulst. Eine dreimalige Wiederholung des Experiments führte zu vergleichbaren Ergebnissen.

### Beispiel 5: Eine Immobilisierung des CD137-Proteins ist notwendig für die Induktion der Monozyten-Proliferation

10⁵ periphere Monozyten wurden über immobilisiertem Fc und immobilisiertem CD137-Fc und zum Vergleich dazu in Gegenwart von Fc und CD137-Fc in gelöster Form kultiviert. Eine Immobilisierung von CD137 und Fc wurde in den Vergleichsansätzen dadurch verhindert, daß man die Kulturgefäße unspezifisch mit Rinderserumalbumin (Inkubation mit 200µl BSA 0,1% ig, 30 min. bei Raumtemperatur) blockierte.

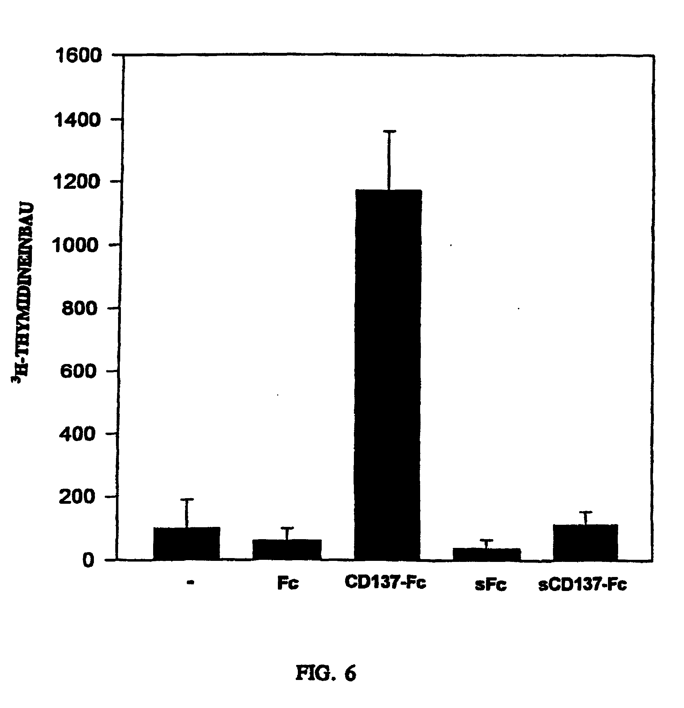

Die Proliferation wurde am Tag 10 über den ³H-Thymidin-Einbau bestimmt. Wie die in Fig. 6 dargestellten Ergebnisse zeigen, war eine Induktion der Monozytenproliferation durch CD137 nur dann zu beobachten, wenn CD137-Protein durch Beschichtung von Gewebekulturschalen mit dem Protein vorher immobilisiert wurde. Wurde dagegen CD137 als lösliches Protein verabreicht, so wurde auch die Induktion der Proliferation deutlich verringert. In drei unabhängigen Experimenten wurden drei vergleichbare Ergebnisse erzielt.

### Beispiel 6: Untersuchung der Monozyten-Proliferation in Gegenwart von TNFR-Fc und Anti-CD68.

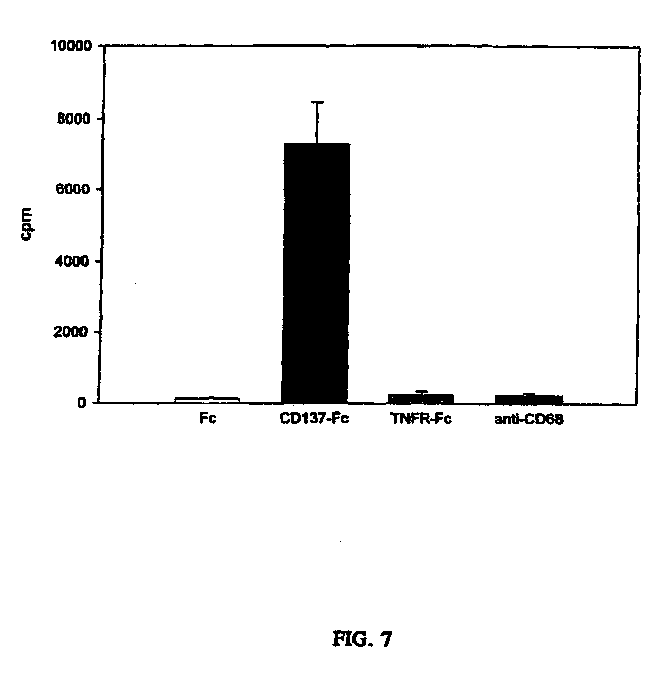

Zur weiteren Charakterisierung der Induktion der Monozyten-Proliferation wurde Fc-Protein, CD137-Fc-Protein, Tumornekrosefaktorrezeptor (TNFR)-Fc-Protein sowie Anti-CD68 immobilisiert. Der ³H-Thymidineinbau wurde, wie oben beschrieben, bestimmt. Aus Fig. 7 wird ersichtlich, daß lediglich CD137-Fc die Monozyten-Proliferation in signifikanter Weise induziert.

TNFR-Fc und Anti-CD68 sind beides Proteine, welche ähnlich wie CD137 an die Oberfläche von Monozyten binden. Mit deren Verwendung als Kontrolle soll ausgeschlossen werden, dass alleine die Bindung von Monozyten an die Oberfläche des Kulturgefäßes die beobachteten Effekte auslöst.

### Beispiel 7: CD137 und M-CSF induzieren in additiver Weise Monozytenproliferation.

10⁵ Monozyten wurden je Vertiefung einer 96-Loch-Mikrotiterplatte vorgelegt. Jede Vertiefung war zuvor mit Fc bzw. CD137-Fc (jeweils 1µg/ml) beschichtet worden. M-CSF (100µg/ml) und LPS (Lipopolysaccharid) (50µg/ml) wurden in gelöster Form zugesetzt. Die Messung der Proliferation nach 8 Tagen Kultivierung erfolgte über den ³H-Thymidineinbau nach einem 24-stündigem Puls mit 0,5µCi ³H-Thymidin.

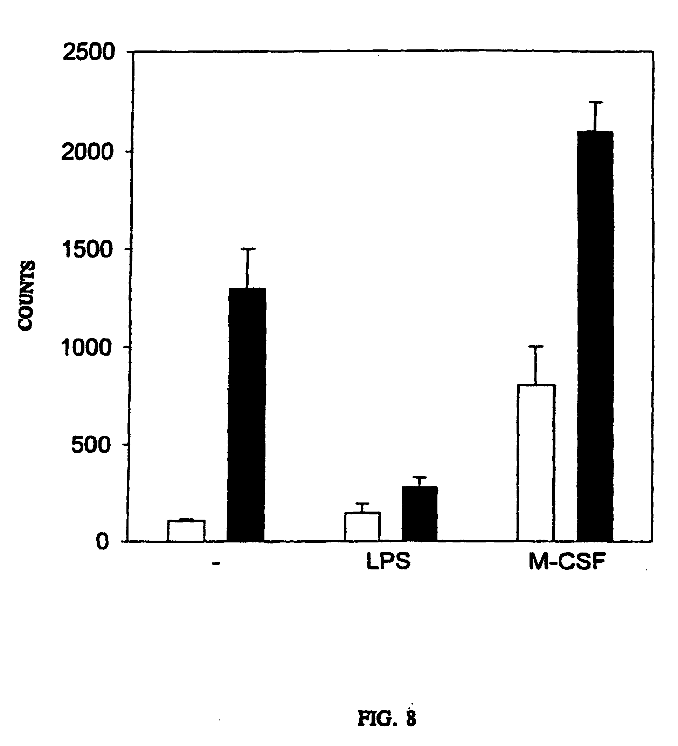

Wie die in Fig. 8 dargestellten Versuchsergebnisse zeigen, wird der ³H-Thymidineinbau in Monozyten durch immobilisiertes CD137-Fc bzw. M-CSF in additiver Weise verstärkt. LPS führt hingegen zu keiner signifikanten Erhöhung der Einbaurate, hat also keinen Effekt auf die CD137-induzierte Monozytenproliferation.

### Beispiel 8: CD137-Fc induziert Morphologische Veränderungen der Monozyten

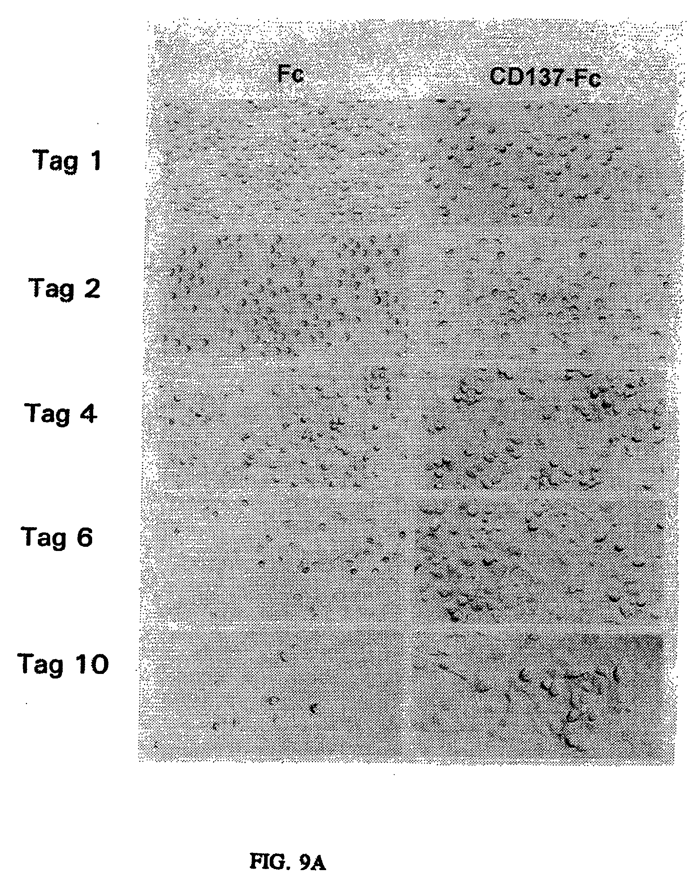

10⁵ primäre periphere Monozyten wurden auf unbehandelten Gewebekulturplatten oder über immobilisiertem Fc oder CD137-Fc-Protein (1 µg/ml) kultiviert. Die Kulturen wurden am Tag 1, 2, 4, 6 und 10 bei 400-facher Vergrößerung fotografiert (Fig. 9A). Kulturplatten, welche lediglich mit Fc-Protein behandelt worden waren, zeigten nur eine leichte Adhäsion der Monozyten. Außerdem behielten die Monozyten ihre runde Morphologie bei. Im Verlauf von 10 Tagen starben bei diesem Ansatz die Monozyten langsam ab (Fig. 9A, linke Spalte). Ein völlig anderes Ergebnis erhält man dagegen, wenn die Zellen auf Gewebekulturplatten gezüchtet wurden, auf welchem CD137-Fc-Fusionsprotein immobilisiert worden war. CD137-Fc induziert ein Anhaften der Monozyten. Diese Zellen nahmen allmählich eine unregelmäßige Gestalt an. Dieser Effekt war bereits am Tag 1 zu beobachten. Mit zunehmender Kultivierungsdauer verteilten sich die Zellen, nahmen an Größe zu und zeigten eine komplexere Morphologie (Fig. 9A, rechte Spalte).

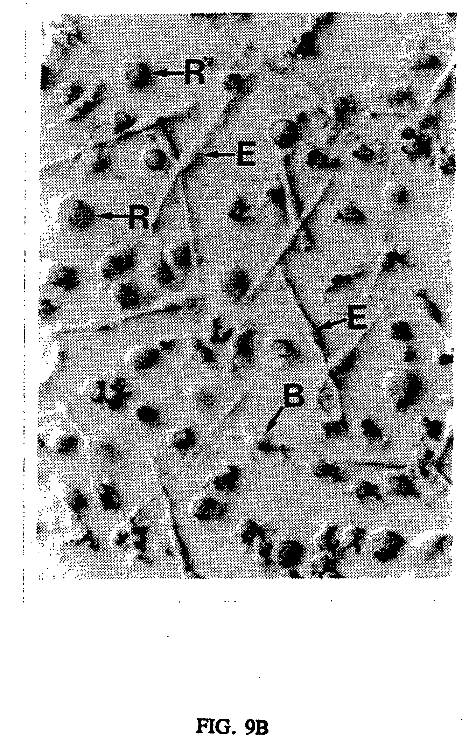

Am Tag 10 der Kultivierung in Gegenwart von immobilisiertem CD137-Fc konnten drei grundlegende Morphologien von in vitro differenzierten Monozyten identifiziert werden: eine elongierte Form (E), eine runde Form (R) und eine verzweigte Form (B) (vgl. Fig. 9B).

### Beispiel 9: CD137-Fc induziert die Bildung von M-CSF

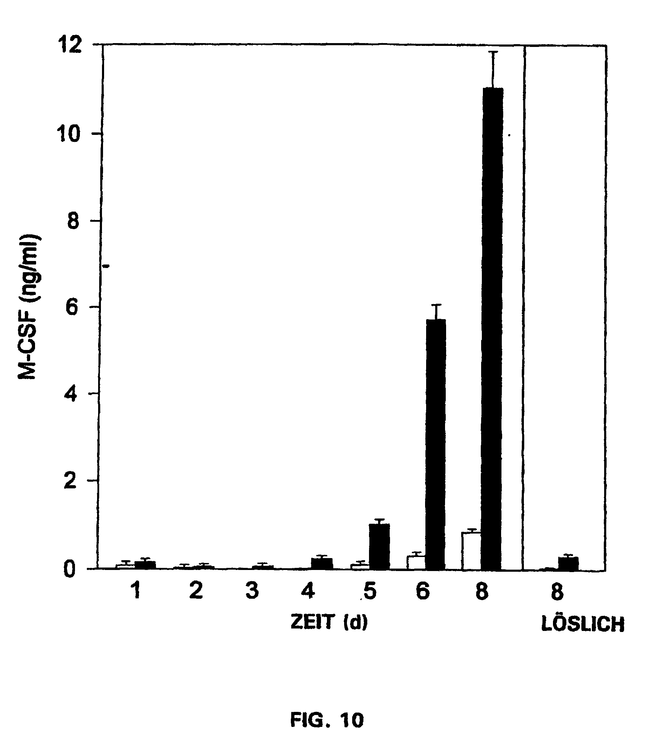

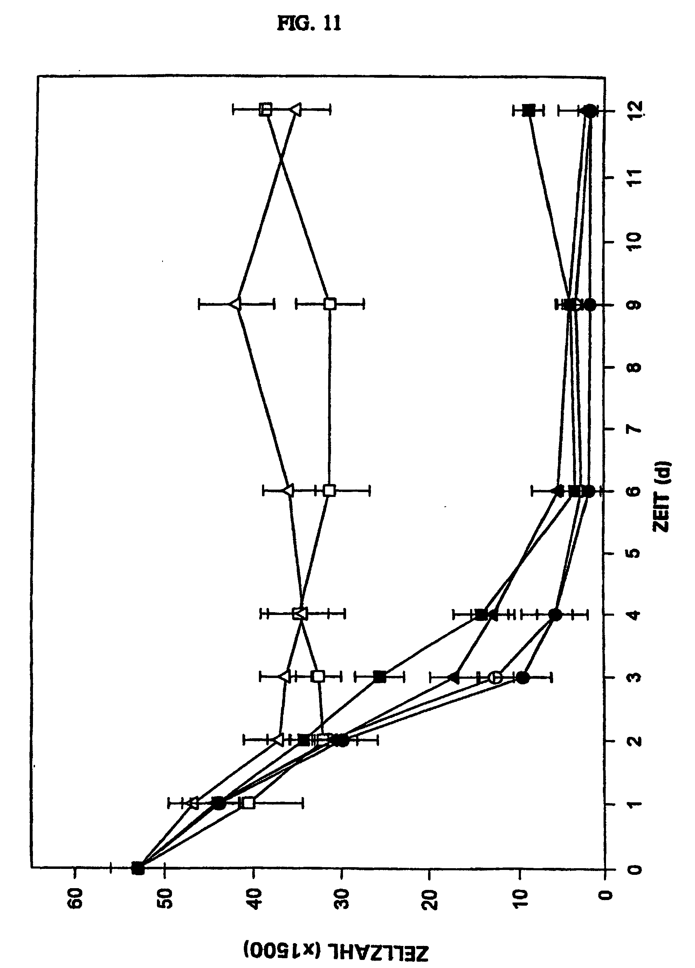

(a) 10⁵ primäre Monozyten wurden in einem ersten Experiment über immobilisiertem Fc oder CD137-Fc (jeweils 1 µg/ml) kultiviert. Um CD137-Fc-Protein (1 µg/ml) in Lösung zu halten, wurde in einem Ansatz die Immobilisierung von CD137-Fc-Protein durch Vorinkubation der Gewebekulturplatten mit fötalem Kälberserum (1 h bei 4°C, unverdünnt) verhindert.
   Die Ergebnisse sind in Fig. 10 dargestellt. Die Kulturüberstände wurden zu den angegebenen Zeiten geerntet und die M-CSF-Konzentration wurde mittels ELISA bestimmt. In drei getrennten Experimenten erhielt man vergleichbare Resultate.
   Wie Fig. 10 zeigt, induziert CD137 die Expression von M-CSF. Während der ersten drei Tage der Kultivierung waren die M-CSF-Gehalte niedrig und es zeigte sich kein Unterschied zwischen der Kontrolle und den CD137-behandelten Monozyten. Ab Tag 4 war die Bildung von M-CSF nachweisbar und die Konzentration von M-CSF nahm rasch und stetig zu. Eine Immobilsierung des CD137-Proteins war notwendige Voraussetzung für eine signifikante M-CSF-Bildung, da lösliches CD137 nur niedrige M-CSF-Konzentrationen induzierte.
(b) In einem nächsten Experiment wurde gezeigt, daß die Expression von M-CSF nicht nur durch CD137 induziert wird, sondern auch für das Überleben CD137-induzierter Monozyten essentiell ist. Hierzu wurden die Monozyten in Gegenwart von immobilisiertem CD137-Protein und neutralisierendem Anti-M-CSF-Antikörper kultiviert. Es wurde beobachtet, daß etwa zehnmal mehr Monozyten auf CD137-Fc-beschichteten Platten im Vergleich zu Fc-Protein-beschichteten Platten überlebten. Die Zugabe neutralisierender Anti-M-CSF-Antikörper verringerte die Zahl überlebender Monozyten auf den Grundwert (vgl. Tabelle 1). Bei Beobachtung des zeitlichen Verlaufs stellte man fest, daß die Mehrzahl der kultivierten Zellen auf unbeschichteten oder Fc-beschichteten Kulturplatten bereits am Tag 6 abgestorben waren (vgl. Fig. 11). Immobilisiertes CD137-Protein oder M-CSF stabilisierten dagegen auch noch am Tag 12 die Zellzahl auf hohem Niveau. Nach Abfangen von M-CSF mit Hilfe neutralisierender Antikörper wurde dieser Effekt nicht mehr beobachtet.

Es ist bekannt, daß neben M-CSF auch GM-CSF und IL-3 im Menschen die Lebensdauer von Monozyten positiv beeinflussen. In den erfindungsgemäßen Experimenten konnte im ELISA keine Expression von GM-CSF oder IL-3 nach 8-tägiger Kultivierung (im Kontrollansatz sowie bei CD137-behandelten Zellen) beobachtet werden (Ergebnisse nicht gezeigt). Neutralisierende Anti-GM-CSF-Antikörper verringerten jedoch die CD137-vermittelte Monozytenüberlebensrate um die Hälfte (vgl. Tabelle 1). Anti-Il-3-Antikörper hatten keinen Effekt. Eine Kombination von neutralisierenden Anti-IL-3-Antikörpern und Anti-GM-CSF-Antikörpern verringerten jedoch die Anzahl überlebender Monozyten nahezu auf den Grundwert. Dies zeigt, daß auch IL-3 eine wichtige Rolle für das Überleben der Monozyten spielt. Eine Neutralisation von GM-CSF und/oder IL-3 zusätzlich zur Neutralisation von M-CSF führt zu keiner weiteren Verringerung der Zellzahl.

Der positive Effekt von M-CSF konnte durch Verabreichung neutralisierender Anti-M-CSF-Antikörper völlig und durch eine Kombination von Anti-GM-CSF- und Anti-IL-3-Antikörpern nahezu auf den Grundwert verringert werden.

**TABELLE 1**

| Stimulus | Neutralisierender Antikörper²⁾ | Anzahl lebender Zellen¹⁾ | p-Wert⁴⁾ |
|---|---|---|---|
| Fc | --- | 6.8 ± 1.5 | --- |
| CD137-Fc | --- | 75.5 ± 6.8 | --- |
| CD137-Fc | α-M-CSF | 9.3 ± 2.0 | 0.005 |
| CD137-Fc | α-IL-3 | 72.3 ± 8.4 | n.s. |
| CD137-Fc | α-GM-CSF | 36.0 ± 2.5 | 0.006 |
| CD137-Fc | α-M-CSF + α-IL-3 | 7.0 ± 1.8 | 0.005 |
| CD137-Fc | α-M-CSF + α-GM-CSF | 6.0 ± 3.0 | 0.007 |
| CD137-Fc | α-GM-CSF + α-IL-3 | 19.0 ± 3.0 | 0,005 |
| CD137-Fc | α-GM-CSF + α-GM-CSF + α-IL-3 | 4.8 ± 3.3 | 0.008 |
| M-CSF | --- | 68.3 ± 16.2 | -- |
| M-CSF | α-M-CSF | 5.3 ± 2.6 | 0.006 |
| M-CSF | α-GM-CSF + α-IL-3 | 20.8 ± 5.0 | 0.018 |

| | | | |
|---|---|---|---|
| ¹⁾ Primäre, periphere Monozyten wurden auf immobilisertem Fc-Protein oder CD137-Fc-Protein (1 µg/ml) kultiviert oder M-CSF wurde in einer Endkonzentration von 100 ng/ml zugesetzt. | | | |
| ²⁾ Die neutralisierenden Antikörper wurden in einer Konzentration von 2 µg/ml verwendet; dessen Zugabe erfolgte zu Beginn des Experiments. ³⁾ Mittlere Zellzahl und Standardabweichung. Am Tag 10 wurden die Zellen in vier repräsentativen Feldern der Platte gezählt. | | | |
| ⁴⁾ Die p-Werte wurden nach dem Student's t-Test mit Hilfe der SSPS-software bestimmt. n.s. = nicht signifikant. | | | |

### Beispiel 10: Eine Immobilisierung von CD137-Protein erhöht die Anzahl lebender Monozyten

10⁵ periphere Monozyten wurden auf immobilisiertem Fc- oder CD137-Fc-Protein (jeweils 1 µg/ml) oder in Gegenwart von löslichem Fc- oder löslichem CD137-Fc-Protein (jeweils 1 µg/ml) kultiviert. Die Kulturen wurden am Tag 8 bei 300-facher Vergrößerung fotografiert (vgl. Fig. 12). Die Immobilisierung erfolgte durch Vorbehandlung der Kulturplatten mit Rinderserumalbumin (30 min Raumtemperatur, 200µl je Vertiefung einer 96-Loch-Platte). In Gegenwart von löslichem Protein ist die Monozytenzahl deutlich verringert. In Übereinstimmung mit diesem Befund wird M-CSF nur dann induziert, wenn CD137-Protein immobilisiert vorliegt (vgl. Beispiel 9). Es ist deshalb wahrscheinlich, daß CD137 auf Monozyten nur dann wirkt, wenn ein von den Monozyten exprimierter CD137-Ligand (d.h. ein Bindungspartner für CD137) über die Bindung von immobilisierten CD137-Molekülen vernetzt wird.

Lösliches CD137-Fc, das über Anti-Fc-Antikörper sekundär vernetzt wird, verlängert ebenfalls das Überleben von Monozyten, beobachtet nach 8-tägiger Kultivierung (Ergebnisse nicht gezeigt). Für dieses Experiment war CD137-Fc (2µg/ml) zuvor mit Ziege-Anti-human-Fc-Antikörper (2µg/ml) vernetzt worden.

## Patentansprüche

1. Verwendung des Monozytenwachstumsfaktors CD137 oder eines funktionalen Analogons davon zur Herstellung eines Medikaments zur Förderung der Proliferation peripherer Monozyten eines Säugers.

2. Verwendung des Monocytenwachstumsfaktors CD137 oder eines funktionalen Analogons davon zur Herstellung eines Medikaments zur Behandlung einer Erkrankung, die mit einer Störung eines Zellsystems assoziiert ist, das Monozyten und/oder davon abgeleiteten Zellen und/oder Progenitoren und/oder Präkursoren davon umfasst; oder deren Entstehung und/oder Verlauf durch Förderung der Proliferation von Zellen dieses Zellsystems therapierbar ist.

3. Verwendung nach Anspruch 2, wobei die Störung des Zellsystems eine Verringerung oder funktionelle Störung peripherer Monozyten und/oder davon abgeleiteter Zellen umfasst.

4. Verwendung nach Anspruch 2, zur Behandlung von Erkrankungen, die mit einer unzureichenden Immunantwort assoziiert sind.

5. Verwendung nach einem der Ansprüche 2 bis 4, wobei die Erkrankung ausgewählt ist unter
a) Chemotherapie- oder Strahlentherapie-bedingten Schädigungen des hämatopoetischen Systems;
b) Wundheilungsstörungen;
c) Tumorerkrankungen;
d) Bakterien-, Virus- oder Pilzinfektionen;
e) angeborene oder nicht-angeborene, erbliche oder nichterbliche Schädigungen oder Erkrankungen des Immunsystems; und
f) durch Behandlung mit Immunsuppressiva induzierte Schädigungen.

6. Verwendung nach einem der vorhergehenden Ansprüche, wobei eine in vivo- oder ex vivo-Behandlung durchgeführt wird.

7. Verwendung nach einem der vorhergehenden Ansprüche, wobei CD137 oder ein funktionales Analogon davon in Kombination mit wenigsten einem weiteren Faktor eingesetzt wird, der ausgewählt ist unter Interleukinen, Lymphokinen, Monokinen, Interferonen, koloniestimulierenden Faktoren und Wachstumsfaktoren.

8. Verwendung nach Anspruch 7, wobei der weitere Faktor ein Leukozyten-stimulierender Faktor ist.

9. Verwendung nach Anspruch 8, wobei der Faktor ausgewählt ist unter G-CSF, GM-CSF und M-CSF.

10. Verwendung nach einem der vorhergehenden Ansprüche, wobei CD137 oder dessen funktionales Analogon in immobilisierter Form oder als multimeres Aggregat eingesetzt wird.

11. Verwendung nach Anspruch 10, wobei das multimere CD137-Aggregat zwei bis fünf CD137-Proteinmoleküle oder funktionale Analoga davon umfasst.

12. Verwendung nach Anspruch 10 oder 11, wobei der extrazelluläre Abschnitt von humanem CD137 entsprechend den Aminosäuren + 18 bis + 186 gemäß Figur 1 A oder ein zur Bindung an Monozyten befähigtes funktionales Analogon davon verwendet wird.

13. In vitro-Verfahren zur Förderung der Proliferation peripherer Monozyten, wobei man periphere Monozyten aus dem Blut eines Säugers in einem Nährmedium mit CD137 in Kontakt bringt und inkubiert, bis die Monozytenzahl und/oder -grösse zunimmt.

14. Verwendung einer Nukleotidsequenz, welche für CD137 oder ein funktionales Analogon davon kodiert, zur Herstellung eines gentherapeutischen Mittels zur Behandlung einer der in einem der Ansprüche 2 bis 5 definierten Erkrankungen.

## Claims

1. Use of the monocyte growth factor CD137 or a functional analogue thereof for preparing a medicament for promoting the proliferation of peripheral monocytes of a mammal.

2. Use of the monocyte growth factor CD137 or a functional analogue thereof for preparing a medicament for treating a disease associated with a disorder of a cell system which comprises monocytes and/or cells derived therefrom and/or progenitors and/or precursors thereof; or whose genesis and/or progress can be treated by promoting the proliferation of cells of this cell system.

3. Use according to claim 2, wherein the disorder of the cell system comprises a reduction in or functional disruption of peripheral monocytes and/or cells derived therefrom.

4. Use according to claim 2, for treating diseases associated with an inadequate immune response.

5. Use according to one of claims 2 to 4, wherein the disease is selected from among:
a) chemotherapy- or radiotherapy-induced damage to the haematopoietic system;
b) wound healing disorders;
c) tumour diseases;
d) bacterial, viral or fungal infections;
e) congenital or non-congenital, inherited or non-inherited damage to or diseases of the immune system; and
f) damage induced by treatment with immunosuppressants.

6. Use according to one of the preceding claims, wherein a treatment is carried out *in vivo* or *ex vivo.*

7. Use according to one of the preceding claims, wherein CD137 or a functional analogue thereof is used in conjunction with at least one additional factor which is selected from among interleukins, lymphokines, monokines, interferons, colony-stimulating factors and growth factors.

8. Use according to claim 7, wherein the additional factor is a leukocyte-stimulating factor.

9. Use according to claim 8, wherein the factor is selected from among G-CSF, GM-CSF and M-CSF.

10. Use according to one of the preceding claims, wherein CD137 or a functional analogue thereof is used in immobilised form or as a multimeric aggregate.

11. Use according to claim 10, wherein the multimeric CD137 aggregate comprises two to five CD137 protein molecules or functional analogues thereof.

12. Use according to claim 10 or 11, wherein the extracellular part of human CD137 corresponding to the amino acids + 18 to + 186 according to Figure 1 A or a functional analogue thereof capable of binding to monocytes is used.

13. *In vitro* process for promoting the proliferation of peripheral monocytes, wherein peripheral monocytes from the blood of a mammal are brought into contact with CD137 in a nutrient medium and incubated until the number and/or size of the monocytes increases.

14. Use of a nucleotide sequence which codes for CD137 or a functional analogue thereof, for preparing a gene therapy agent for treating one of the diseases defined in one of claims 2 to 5.

## Revendications

1. Utilisation du facteur de croissance de monocytes CD137 ou d'un analogue fonctionnel de celui-ci pour fabriquer un médicament pour stimuler la prolifération de monocytes périphériques chez un mammifère.

2. Utilisation du facteur de croissance de monocytes CD137 ou d'un analogue fonctionnel de celui-ci dans la fabrication d'un médicament pour le traitement d'une maladie associée à un trouble d'un système cellulaire, qui comprend des monocytes et/ou des cellules dérivées de ceux-ci et/ou des progéniteurs et/ou des précurseurs de ceux-ci, ou dont l'apparition et/ou l'évolution peut être traitée par activation de la prolifération de cellules appartenant audit système cellulaire.

3. Utilisation selon la revendication 2, dans les cas où le trouble du système cellulaire comprend une diminution ou un trouble fonctionnel de monocytes périphériques et/ou de cellules dérivées de ceux-ci.

4. Utilisation selon la revendication 2 pour le traitement de maladies associées à une réponse immunitaire insuffisante.

5. Utilisation selon une des revendications 2 à 4, dans les cas de maladie suivants:
a) lésions du système hématopoïétique consécutives à des traitements par chimiothérapie ou par radiothérapie;
b) troubles du processus de cicatrisation;
c) maladies tumorales;
d) infections d'origine bactérienne, virale ou fongique;
e) lésions congénitales ou non, graves ou non, ou maladies du système immunitaire; et
f) lésions induites par des traitements par immuno-suppresseurs.

6. Utilisation selon une des revendications précédentes, dans laquelle un traitement est réalisé in vivo ou ex vivo.

7. Utilisation selon une des revendications précédentes, dans laquelle on applique le facteur CD137 ou un analogue fonctionnel de celui-ci en combinaison avec au moins un facteur supplémentaire sélectionné parmi les interleukines, le lymphokines, les monokines, les interférons, les facteurs de stimulation de colonies et les facteurs de croissance.

8. Utilisation selon la revendication 7, dans laquelle le facteur supplémentaire est un facteur de stimulation des leucocytes.

9. Utilisation selon la revendication 8, dans laquelle le facteur est choisi parmi les facteurs G-CSF, GM-CSF et M-CSF.

10. Utilisation selon une des revendications précédentes, dans laquelle on utilise le facteur CD137 ou un analogue fonctionnel de celui-ci sous forme immobilisée ou comme multimère agrégé.

11. Utilisation selon la revendication 10, dans laquelle le multimère agrégé de CD137 contient de deux à cinq molécules de protéine CD137 ou des analogues fonctionnels de celles-ci.

12. Utilisation selon la revendication 10 ou 11, dans laquelle on utilise la partie extra-cellulaire de CD137 humain correspondant aux acides aminés +18 à +186 selon la figure 1A ou un analogue fonctionnel de ceux-ci pouvant se lier aux monocytes.

13. Procédé in vitro pour stimuler la prolifération de monocytes périphériques, dans lequel on met des monocytes périphériques du sang d'un mammifère en contact avec le facteur CD137 dans un milieu de culture et on le fait incuber jusqu'à ce que le nombre et/ou la taille des monocytes augmente.

14. Utilisation d'une séquence de nucléotides codée pour le facteur CD137 ou un analogue fonctionnel de celui-ci pour fabriquer un produit de génothérapie pour le traitement de l'une des maladies définies dans l'une des revendications 2 à 5.
